(19) Europäisches Patentamt
European Patent Office
Office européen des brevets

(11) EP 4 567 049 A1

(12) EUROPEAN PATENT APPLICATION
published in accordance with Art. 153(4) EPC

(43) Date of publication:
**11.06.2025 Bulletin 2025/24**

(21) Application number: **23849805.9**

(22) Date of filing: **29.06.2023**

(51) International Patent Classification (IPC):
***C08B 31/10*** (2006.01) ***A61F 13/53*** (2006.01)
***A61K 47/36*** (2006.01) ***A61L 27/20*** (2006.01)
***B01J 20/26*** (2006.01) ***A23L 5/00*** (2016.01)
***A61L 15/22*** (2006.01)

(52) Cooperative Patent Classification (CPC):
**A23L 5/00; A61F 13/53; A61K 47/36; A61L 15/22; A61L 27/20; B01J 20/26; C08B 31/10**

(86) International application number:
**PCT/JP2023/024166**

(87) International publication number:
**WO 2024/029246 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.08.2022 JP 2022122452**

(71) Applicants:
- **Nagase & Co., Ltd.**
  **Osaka-shi, Osaka 550-8668 (JP)**
- **NAGASE CHEMTEX CORPORATION**
  **Osaka-shi, Osaka 550-8668 (JP)**
- **Nagase Viita Co., Ltd.**
  **Okayama-shi, Okayama 702-8006 (JP)**

(72) Inventors:
- **NOZAKI, Takahiro**
  **Tokyo 100-8142 (JP)**
- **TANAKA, Atsushi**
  **Tokyo 100-8142 (JP)**
- **NISHIMOTO, Tomoyuki**
  **Kobe-shi, Hyogo 651-2241 (JP)**
- **HOSOMI, Tetsuya**
  **Tatsuno-shi, Hyogo 679-4124 (JP)**
- **MIYATA, Manabu**
  **Okayama-shi, Okayama 702-8006 (JP)**

(74) Representative: **Mewburn Ellis LLP**
**Aurora Building**
**Counterslip**
**Bristol BS1 6BX (GB)**

# (54) ABSORBENT ARTICLE

(57) The present invention provides an absorbent article that contains a water-absorbing resin having excellent water absorption performance. The present invention relates to an absorbent article containing a water-absorbing resin including a water-soluble polymer obtained by introducing an acidic group into a starch or a partially degraded product thereof.

## Description

TECHNICAL FIELD

The present invention relates to an absorbent article.

BACKGROUND ART

Absorbent articles are used in the fields of hygiene and medicine, for example, as disposable diapers, sanitary products, and wound protection materials and are also used in food, agriculture and forestry, civil engineering, and other fields. These absorbent articles contain water-absorbing resins to be able to absorb liquids. Partially neutralized salts of polyacrylic acids or polymethacrylic acids are widely used as such water-absorbing resins. Also known are water-absorbing resins made from polysaccharides such as starch.

Patent Literature 1 discloses a method for producing a water-absorbing resin by heat drying a carboxyalkylated starch to cross-link the starch molecules. This document describes that a decrease in the molecular weight of the starch is preferably reduced during the carboxyalkylation reaction.

Patent Literature 2 discloses a method for producing a water-absorbing resin by subjecting carboxyalkylated polysaccharide particles to surface treatment with a non-cross-linking acid such as hydrochloric acid, followed by heat drying or an interaction with a cross-linking agent to cross-link the polysaccharide particles.

Patent Literature 3 discloses a method for producing a water-absorbing material by reacting a starch with a polybasic acid anhydride in an extruder. This document describes that a decrease in the molecular weight of the starch is preferably reduced during the reaction with the polybasic acid anhydride.

CITATION LIST

- Patent Literature

Patent Literature 1: U.S. Patent No. 5079354
Patent Literature 2: JP 2010-504414 T
Patent Literature 3: JP 2007-222704 A

SUMMARY OF INVENTION

- Technical Problem

Conventional water-absorbing resins made from polysaccharides do not have sufficient water absorption performance. The present invention aims to provide an absorbent article that contains a water-absorbing resin having excellent water absorption performance.

- Solution to Problem

The present inventors made studies on the chemical structure of water-absorbing resins made from polysaccharides and found a water-absorbing resin having excellent water absorption performance. The inventors used this water-absorbing resin in absorbent articles, thereby completing the present invention.

Specifically, the present invention relates to an absorbent article, containing a water-absorbing resin including a water-soluble polymer obtained by introducing an acidic group into a starch or a partially degraded product thereof.

The acidic group is preferably a carboxyalkyl group, a carboxyalkenyl group, or a sulfoalkyl group.

The starch or partially degraded product thereof preferably has at least one of a weight average molecular weight (Mw) of 7,500,000 or less or a dispersity (weight average molecular weight (Mw)/number average molecular weight (Mn)) of 5 or more.

The water-soluble polymer preferably has a weight average molecular weight (Mw) of 500,000 to 40,000,000 as determined by aqueous size exclusion chromatography using pullulan standards.

The water-absorbing resin preferably has at least one of the following characteristics:

(a) a water absorbency under no pressure for ion-exchanged water is 100 to 500 g/g;
(b) a water retention rate for ion-exchanged water is 80 to 300 g/g;

(c) a water absorbency under no pressure for physiological saline is 10 to 70 g/g; and
(d) a water retention rate for physiological saline is 5 to 60 g/g.

The absorbent article is preferably at least one selected from the group consisting of hygiene supplies, medical supplies, food storage supplies, civil engineering supplies, and agricultural and horticultural supplies.
The hygiene supplies are preferably at least one selected from the group consisting of disposable diapers, sanitary products, incontinence pads, breast pads, portable toilets, and pet toilets.
The medical supplies are preferably at least one selected from the group consisting of hemostatic sponges, wound protection materials, wound healing materials, medical waste solidifiers, cold packs, drug delivery system carriers, and artificial joints.
The present invention also relates to a recycled water-soluble polymer, obtained by treating the absorbent article with an alkali.
The present invention also relates to a water-absorbing resin that is a cross-linked product of the recycled water-soluble polymer.
The present invention also relates to a water-absorbing article, containing the water-absorbing resin.
The present invention also relates to a method for recovering hydrophilic fibers from an absorbent article containing a water-absorbing resin and the hydrophilic fibers, the water-absorbing resin including a water-soluble polymer obtained by introducing an acidic group into a starch or a partially degraded product thereof, the method including the step of degrading the water-absorbing resin by treating a part or an entirety of the water absorbent article with an alkali.

- Advantageous Effects of Invention

The water absorbent article of the present invention has excellent water absorption performance. Moreover, it contains a water-absorbing resin made from a polysaccharide, which can be degraded and recycled, thereby reducing the environmental burden.

BRIEF DESCRIPTION OF DRAWING

FIG. 1 shows the results of gel filtration chromatography of water-soluble polymers.
FIG. 2 shows infrared absorption spectra of the water-soluble polymers.

DESCRIPTION OF EMBODIMENTS

<Absorbent article>

The absorbent article of the present invention is characterized by containing a water-absorbing resin including a water-soluble polymer obtained by introducing an acidic group into a starch or a partially degraded product thereof.

<Starch or partially degraded product thereof>

The present invention uses a starch or a partially degraded starch as a main material of the water-absorbing resin. The use of a starch allows the resulting water-absorbing resin to be highly safe for the human body, to have excellent water absorption, and to have a low environmental burden at the time of disposal. The type of starch as a raw material is not limited, and examples include tapioca starch, potato starch, corn starch such as waxy corn starch and high amylose starch, wheat starch, rice starch, and sweet potato starch. The term "partially degraded starch" refers to a product obtained by breaking some of the glucoside bonds of a starch, i.e., a polymer in which a large number of $\alpha$-glucose molecules are linked via glucoside bonds, although the breakage position and mode are not limited. In the method for producing the water-absorbing resin, which will be described later, a starch and a partially degraded starch may be used in combination.
When using a partially degraded starch, the weight average molecular weight (Mw) of the partially degraded starch is preferably, but not limited to, 7,500,000 or less, more preferably 5,000,000 or less, still more preferably 4,500,000 or less, further preferably 4,000,000 or less. The lower limit of the weight average molecular weight of the partially degraded starch is preferably, but not limited to, 50,000 or more, more preferably 100,000 or more, still more preferably 200,000 or more. If the weight average molecular weight is less than 50,000, the water absorption amount of the water-absorbing resin tends to decrease. In the case where the viscosity of the starch used affects operability in the production, two or more starches and/or partially degraded starches with different weight average molecular weights or other additives, etc. can be used in combination, depending on the application or cost. Here, the weight average molecular

weight can be measured by any method. For example, it may be determined by aqueous size exclusion chromatography relative to a calibration curve of molecular weight vs. elution time prepared using pullulans with known molecular weights.

The dispersity (weight average molecular weight (Mw)/number average molecular weight (Mn)) of the partially degraded starch is generally, but not limited to, 5 or more. If the dispersity is less than 5, the water absorption amount of the water-absorbing resin tends to decrease. The upper limit of the dispersity is not limited. The number average molecular weight can be measured by any method. For example, it may be determined by aqueous size exclusion chromatography relative to a calibration curve of molecular weight vs. elution time prepared using pullulans with known molecular weights.

The weight average molecular weight and dispersity of the starch or partially degraded product thereof are as described above, but when the starch is used alone, it is preferably a starch derived from waxy corn, tapioca, corn, or potato, more preferably a starch derived from waxy corn, tapioca, or corn. When the partially degraded starch is used alone, the weight average molecular weight thereof is preferably 50,000 to 7,500,000, more preferably 50,000 to 5,000,000, still more preferably 100,000 to 4,000,000. Also, the dispersity is preferably 5 or more, more preferably 7 or more.

<Water-soluble polymer, Acidic group>

As used herein, the term "water-soluble polymer" refers to a polymer obtained by introducing an acidic group into a starch or a partially degraded product thereof.

Examples of the acidic group include carboxyl group-containing acidic groups such as carboxyalkyl and carboxyalkenyl groups; sulfo group-containing acidic groups such as sulfoalkyl and sulfoalkenyl groups; and phospho group-containing acidic groups such as phosphoalkyl and phosphoalkenyl groups.

A carboxyalkyl group refers to an alkyl group substituted with a carboxyl group. The number of carbon atoms of the alkyl group to be substituted with a carboxyl group is preferably 1 to 8, more preferably 1 to 5. The alkyl group may be either linear or branched. Specific examples of the alkyl group include a methyl group, an ethyl group, an n-propyl group, an i-propyl group, an n-butyl group, an i-butyl group, an s-butyl group, a t-butyl group, an n-pentyl group, a 1-methyl-n-butyl group, a 2-methyl-n-butyl group, a 3-methyl-n-butyl group, a 1,1-dimethyl-n-propyl group, a 1,2-dimethyl-n-propyl group, a 2,2-dimethyl-n-propyl group, and a 1-ethyl-n-propyl group.

Specific examples of such carboxyalkyl groups include a carboxymethyl group, a carboxyethyl group, a carboxypropyl group, a carboxybutyl group, and a carboxypentyl group.

A carboxyalkenyl group refers to an alkenyl group substituted with a carboxyl group. The number of carbon atoms of the alkenyl group to be substituted with a carboxyl group is preferably 2 to 8, more preferably 2 to 4. The alkenyl group may be either linear or branched. Specific examples of the alkenyl group include an ethenyl group, a 1-propenyl group, a 2-propenyl group, a 1-methyl-1-ethenyl group, a 1-butenyl group, a 2-butenyl group, a 3-butenyl group, a 2-methyl-1-propenyl group, a 2-methyl-2-propenyl group, a 1-ethylethenyl group, a 1-methyl-1-propenyl group, and a 1-methyl-2-propenyl group.

Specific examples of such carboxyalkenyl groups include a carboxyethenyl group, a carboxypropenyl group, and a carboxybutenyl group.

A sulfoalkyl group refers to an alkyl group substituted with a sulfo group. Examples of the alkyl group to be substituted with a sulfo group include the alkyl groups listed for the carboxyalkyl groups. Specific examples of such sulfoalkyl groups include a sulfomethyl group, a sulfoethyl group, and a sulfopropyl group.

A sulfoalkenyl group refers to an alkenyl group substituted with a sulfo group. Examples of the alkenyl group to be substituted with a sulfo group include the alkenyl groups listed for the carboxyalkenyl groups. Specific examples of such sulfoalkenyl groups include a sulfoethenyl group and a sulfopropenyl group.

A phosphoalkyl group refers to an alkyl group substituted with a phospho group. Examples of the alkyl group to be substituted with a phospho group include the alkyl groups listed for the carboxyalkyl groups. Specific examples of such phosphoalkyl groups include a phosphomethyl group, a phosphoethyl group, and a phosphopropyl group.

A phosphoalkenyl group refers to an alkenyl group substituted with a phospho group. Examples of the alkenyl group to be substituted with a phospho group include the alkenyl groups listed for the carboxyalkenyl groups. Specific examples of such phosphoalkenyl groups include a phosphoethenyl group and a phosphopropenyl group.

In view of ease of introduction into the starch or partially degraded product thereof, the acidic group is preferably a carboxyl group-containing acidic group or a sulfo group-containing acidic group, more preferably a carboxyalkyl group, a carboxyalkenyl group, or a sulfoalkyl group, still more preferably a carboxyalkyl group having 1 to 5 carbon atoms.

In the water-soluble polymer, an acidic group is introduced into a starch or a partially degraded product thereof, and additionally the hydrogen atoms of the hydroxyl groups of the glucose units may be replaced by a functional group other than the acidic group. Examples of the functional group other than the acidic group include hydrocarbon groups such as a methyl group and an ethyl group, and hydroxyl group-containing substituents such as a hydroxypropyl group and a hydroxyethyl group. The position of the hydroxyl group replaced by the functional group other than the acidic group may be any of the 1-, 2-, 3-, **4-,** and 6-positions, preferably the 2-, 3-, or 6-position, of the glucose unit.

The molecular weight of the water-soluble polymer is not limited, but the water-soluble polymer preferably has a weight average molecular weight of 500,000 to 40,000,000, more preferably 800,000 to 35,000,000, as determined by aqueous size exclusion chromatography using pullulan standards. If the weight average molecular weight is less than 500,000, the water absorption performance of the water-absorbing resin tends to decrease, whereas if it is more than 40,000,000, the viscosity tends to be high, thereby reducing handleability in the production. Here, the weight average molecular weight determined by aqueous size exclusion chromatography using pullulan standards can be determined by aqueous size exclusion chromatography relative to a calibration curve of molecular weight vs. elution time prepared using pullulans with known molecular weights.

The total acid value of the water-soluble polymer is preferably 50 to 350 mg KOH/g, more preferably 70 to 300 mg KOH/g. Although some of the salts of the acidic groups added to the starch or partially degraded starch are neutralized during the production of the water-absorbing resin, as described later, the total acid value means the acid value measured by returning the neutralized acidic groups to their unneutralized state, indicating the amount of all acidic groups in the water-soluble polymer. If the total acid value is less than 50 mg KOH/g or more than 350 mg KOH/g, the water-absorbing resin obtained by cross-linking the water-soluble polymer tends to show reduced water absorption performance for an aqueous solution containing an electrolyte such as physiological saline.

When an acidic group is introduced by reacting a haloalkyl group having the acidic group with the hydroxyl group of a starch to prepare a water-soluble polymer, the amount of acidic groups introduced can also be represented by the degree of etherification. The degree of etherification is preferably 0.1 to 2.0, more preferably 0.2 to 1.5. The degree of etherification can be determined by an ashing-titration method, for example. Moreover, since the total acid value is detected due to the introduced acidic groups, when the starch or partially degraded starch used as a raw material contains no acidic group, the acidic groups detected by the measurement of the total acid value are considered to be equivalent to those introduced by the etherification reaction. Therefore, when the starch or partially degraded starch as a raw material contains no acidic group, the degree of etherification may be calculated simply from the total acid value measured. For example, when all carboxymethyl groups, as acidic groups, have been neutralized to sodium salts, the degree of etherification can be calculated as follows:

Degree of etherification = (162 × total acid value) = (56100 - 80 × total acid value).

Here, the total acid value in this case is expressed in units of mg KOH/g.

The free acid value of the water-soluble polymer is preferably 5 to 50 mg KOH/g, more preferably 7 to 25 mg KOH/g. The term "free acid value" means the acid value measured for unneutralized acidic groups. If the free acid value is less than 5 mg KOH/g or more than 50 mg KOH/g, the water absorption performance of the water-absorbing resin tends to decrease.

The dispersity (weight average molecular weight/number average molecular weight) of the water-soluble polymer is preferably, but not limited to, 5 to 110, more preferably 7 to 70. If the dispersity is less than 5 or more than 110, the water absorption performance of the water-absorbing resin tends to decrease. The number average molecular weight of the water-soluble polymer can be determined by aqueous size exclusion chromatography relative to a calibration curve of molecular weight vs. elution time prepared using pullulans with known molecular weights.

<Cross-linked structure of water-absorbing resin>

Generally, a water-absorbing resin containing a polyacrylic acid as a main constitutional unit has a covalent network structure and can form a chemical gel upon absorption of water. In contrast, the water-absorbing resin used in the present invention is characterized by forming a physical gel upon absorption of water. The cross-linking points in the physical gel may relatively easily disappear due to, for example, the thermal motion of the molecular chains or changes in pH or ionic strength, so that the physical gel can be converted into flowable sol. Therefore, the physical gel can be easily degraded by, for example, adding an alkali or an acid, heating, or shaking, which leads to a reduced environmental burden at the time of disposal.

Whether a physical gel is formed can be determined by analyzing the sol-gel transition based on the disappearance of cross-linking points. For example, it may be determined by fluidization after alkali or acid treatment when the gel has been cross-linked via ionic bonds and/or hydrogen bonds. Specifically, a water-absorbing resin can be considered to form a physical gel upon absorption of water if the water-absorbing resin is suspended in a 1% aqueous sodium hydroxide solution to a final concentration of 5% by weight, stirred for 60 minutes, and then sieved through a sieve having an opening of 500 μm, and the dry weight of the water-absorbing resin remaining on the sieve is less than 2% by weight of the dry weight of the water-absorbing resin contained in the aqueous solution.

More specifically, 0.5 g of a water-absorbing resin may be suspended in 9.5 g of a 1% aqueous sodium hydroxide solution, stirred for 60 minutes, and subjected to natural filtration through an about 50 mm × 50 mm square 30-mesh sieve

(opening: 500 μm), followed by washing the top of the mesh with ion-exchanged water. After the washing, the sieve may be dried with a fan dryer at 120°C for two hours. The water-absorbing resin can be considered to form a physical gel upon absorption of water if the 30-mesh pass residue calculated by the following equation is less than 2% by weight, where Wm1 represents the weight of the sieve before filtration and Wm2 represents the weight of the sieve after filtration and drying. Here, the sieve having an opening of 500 μm corresponds to a 30-mesh sieve stipulated in JIS.

$$\text{30-Mesh pass residue (\%)} = (Wm2 - Wm1)/0.5 \times 100$$

The 30-mesh pass residue is preferably less than 2% by weight, more preferably less than 1.5% by weight, still more preferably less than 1.0% by weight, further preferably less than 0.5% by weight.

The water-absorbing resin is a cross-linked product of the water-soluble polymer described above. The cross-links include internal cross-links and optionally surface cross-links. However, the water-absorbing resin preferably has no internal cross-linked structure via ether bonds or ester bonds. The water-absorbing resin also preferably has no internal cross-linked structure via covalent bonds. Examples of the covalent bonds include, in addition to ester bonds and ether bonds, carbon-carbon covalent bonds and double bonds. Examples of the internal cross-linked structure constituting the water-absorbing resin include ionic bonding of the acidic and neutralized acidic groups on the water-soluble polymer; ionic bonding of the water-soluble polymer with a compound having an opposite charge (a compound containing a basic group such as a cationic group or an amino group if the acidic group of the water-soluble polymer is a carboxylic acid); ionic bonding by divalent alkali metal ions; coordination bonding with metal ions; and hydrogen bonding formed by, for example, dimerization of a carboxylic acid if the acidic group used is a carboxylic acid group, etc. The method for forming surface cross-links will be described later.

The water-absorbing resin may optionally contain a constitutional unit other than the water-soluble polymer described above. Examples of the constitutional unit other than the water-soluble polymer include compounds having a charge opposite to that of the water-soluble polymer, such as chitosan, polyethyleneimine, vinylpyrrolidone-N,N-dimethylaminoethyl methacrylic acid copolymers and other amino group-containing polymers or salts thereof, dimethylamine-epichlorohydrin copolymers, polydiallyldimethylammonium chloride and other quaternary ammonium group-containing polymers. When the water-absorbing resin contains a constitutional unit other than the water-soluble polymer, the amount of the constitutional unit is preferably 90% by weight or less, more preferably 50% by weight or less, relative to the combined amount of the constitutional unit and the water-soluble polymer used as the main component.

The water-absorbing resin may contain water and/or a hydrophilic solvent. Examples of the hydrophilic solvent include methanol, ethanol, n-propanol, isopropanol, acetone, ethylene glycol, propylene glycol, diethylene glycol, ethylene glycol monoethyl ether, ethylene glycol monobutyl ether, diethylene glycol dimethyl ether, and dimethylsulfoxide. The amount of water and/or hydrophilic solvent in the water-absorbing resin is preferably 0.1 to 20%, more preferably 1 to 19%. If the amount is less than 0.1%, the water absorption rate tends to decrease, whereas if it is more than 20%, the water-absorbing resin particles tend to easily aggregate upon absorption of water. The amount of water and/or hydrophilic solvent can be adjusted by subjecting the water-absorbing resin to a treatment such as heating or drying.

<Physical properties of water-absorbing resin>

The water absorbency under no pressure of the water-absorbing resin is determined by measuring the absorbency for physiological saline or ion-exchanged water of the water-absorbing resin with no load applied thereto as described in EXAMPLES. The water-absorbing resin used in the present invention in a solid state preferably has a water absorbency under no pressure for ion-exchanged water that is 100 to 500 g/g, more preferably 100 to 400 g/g, still more preferably 120 to 350 g/g. The water-absorbing resin used in the present invention also preferably has a water absorbency under no pressure for physiological saline that is 10 to 70 g/g, more preferably 20 to 65 g/g.

The water-absorbing resin preferably has a ratio (A/B) of the water absorbency under no pressure for ion-exchanged water (A) to the water absorbency under no pressure for physiological saline (B) that is 7 or less, more preferably 5 or less.

The water retention rate of the water-absorbing resin is determined by measuring the absorbency for physiological saline or ion-exchanged water of the water-absorbing resin with a load of 150 G applied thereto as described in EXAMPLES. The water-absorbing resin in the present invention in a solid state preferably has a water retention rate for ion-exchanged water that is 80 to 300 g/g, more preferably 100 to 250 g/g. The water-absorbing resin in the present invention also preferably has a water retention rate for physiological saline that is 5 to 60 g/g, more preferably 10 to 60 g/g, still more preferably 20 to 60 g/g.

<Method for producing water-absorbing resin>

The water-absorbing resin can be produced by any method, as long as the produced water-absorbing resin has

the structure described above. For example, it can be produced through the step of forming an internal cross-linked structure in a starch or a partially degraded product thereof. The method may optionally further include, before the step of forming an internal cross-linked structure, the step of partially degrading the starch and/or the step of introducing an acidic group. In the method including the step of partially degrading the starch and the step of introducing an acidic group, these steps may be performed in any order, and either step may be performed first.

In the step of partially degrading the starch, the $\alpha$-1,4-glucoside bonds of the $\alpha$-glucose molecules constituting the starch are often broken, although the breakage position and mode are not limited. Any breakage method may be used, including, for example, enzymatic treatment, acid treatment, and physical crushing. These methods may be used in combination. The enzymatic treatment may be performed after or at the same time as gelatinization of the starch. The method for performing enzymatic treatment after gelatinization of the starch may include first suspending the starch in water, heating the suspension to gelatinize it, and then adding an enzyme to cause an enzymatic reaction. Moreover, the method for performing enzymatic treatment at the same time as gelatinization of the starch may include suspending the starch in water, adding an enzyme to the suspension to give a mixture, and then heating the mixture in a temperature range that does not completely deactivate the enzyme. In particular, the method is preferably performed while the starch is gelatinized by heating and kneading the starch at 70°C to 110°C.

When the starch is partially degraded by enzymatic treatment, any enzyme that can degrade the starch may be used. The enzyme may be either an exo-type enzyme or an endo-type enzyme. Specific examples of the enzyme include $\alpha$-amylase, amylomaltase, cyclomaltodextrin glucanotransferase, 4-$\alpha$-glucanotransferase, 4,6-$\alpha$-glucanotransferase, neopullulanase, and amylopullulanase. These enzymes may be used in combination. The pH during the enzymatic treatment is preferably, but not limited to, 5.0 to 7.0. The pH can be adjusted by adding hydrochloric acid, acetic acid, sodium hydroxide, potassium hydroxide, or the like.

When the starch is partially degraded by acid treatment, any acid that can degrade the starch may be used. Specific examples include hydrochloric acid, sulfuric acid, oxalic acid, acetic acid, formic acid, and trifluoroacetic acid. The temperature during the acid treatment is preferably 150°C to 160°C.

When the starch is partially degraded by physical crushing, examples of specific means include radiation exposure, shearing, trituration, high-pressure treatment, ultrasonication, heat degradation, photodegradation, and combinations thereof.

The starch or partially degraded product thereof may be a bleached starch or a derivative obtained by replacing the hydrogen atoms of the hydroxyl groups of the glucose units with a functional group (also referred to as a modified starch or a chemically modified starch). The position of the hydroxyl group whose hydrogen atom is replaced may be any of the 1-, 2-, 3-, 4-, and 6-positions, preferably the 2-, 3-, or 6-position, of the glucose unit. Examples of the functional group include hydrocarbon groups such as a methyl group and an ethyl group; hydroxyl group-containing substituents such as a hydroxypropyl group and a hydroxyethyl group; and carboxyl group-containing substituents such as a carboxymethyl group. Examples of the derivative of the starch or partially degraded product thereof include what is called modified starch used in foods or for industrial purposes, such as acetylated distarch adipate, acetylated distarch phosphate, acetylated oxidized starch, starch sodium octenyl succinate, starch acetate, oxidized starch, hydroxypropyl starch, hydroxypropyl distarch phosphate, phosphated distarch phosphate, monostarch phosphate, distarch phosphate, cationized starch, and urea phosphate esterified starch. Methyl-ethylated starch, hydroxypropyl-methylated starch, and other starches substituted with two or more functional groups are also usable. Among these, starch acetate, oxidized starch, hydroxypropyl starch, and starch sodium octenyl succinate are preferred to easily control the properties of the water-absorbing resin.

In the case where the starch or partially degraded product thereof is a derivative obtained by replacing the hydrogen atoms of the hydroxyl groups of the glucose units with an acidic group, the method may further include the step of introducing an acidic group to add the acidic group. Alternatively, the method may include the step of forming an internal cross-linked structure instead of the step of introducing an acidic group.

In the step of introducing an acidic group, the starch or partially degraded starch may be reacted with an acidic group-containing compound or a precursor thereof. This reaction allows the acidic group to be introduced into the hydroxyl group of the starch or partially degraded starch. The acidic group-containing compound is not limited as long as it can introduce the acidic group described above. Examples include acidic group-containing haloalkyl compounds, acidic group-containing haloalkenyl compounds, acid anhydrides, and salts thereof. Examples of the halogens constituting the haloalkyl compounds or haloalkenyl compounds include chlorine and bromine.

Specific examples of the acidic group-containing compound include monochloroacetic acid, monobromoacetic acid, 3-bromopropionic acid, 6-bromohexanoic acid, succinic anhydride, maleic anhydride, vinylsulfonic acid, phosphorus oxychloride, ethyl monochloroacetate, and sodium or potassium salts thereof. Specific examples of these salts include sodium monochloroacetate, potassium monochloroacetate, and sodium vinylsulfonate.

Examples of the precursor of the acidic group-containing compound include acrylonitrile. Acrylonitrile may be used, for example, by first reacting acrylonitrile with the starch or partially degraded product thereof under basic conditions to introduce a cyanoethyl group, deriving an amide group from the cyanoethyl group (Synthesis; 1989(12): 949-950), and then subjecting the resulting amide to alkaline hydrolysis.

The reaction of the starch or partially degraded starch with monochloroacetic acid to produce a water-soluble polymer is summarized in formula (I).

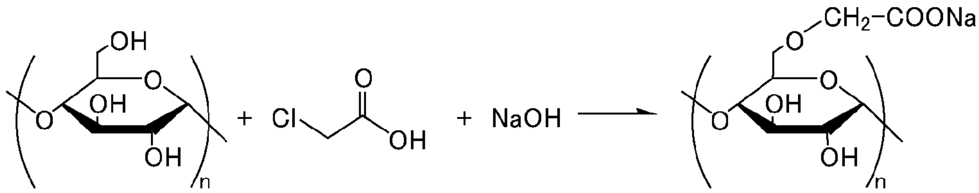

(I)

The reaction of the starch or partially degraded starch with 3-bromopropionic acid to produce a water-soluble polymer is summarized in formula (II).

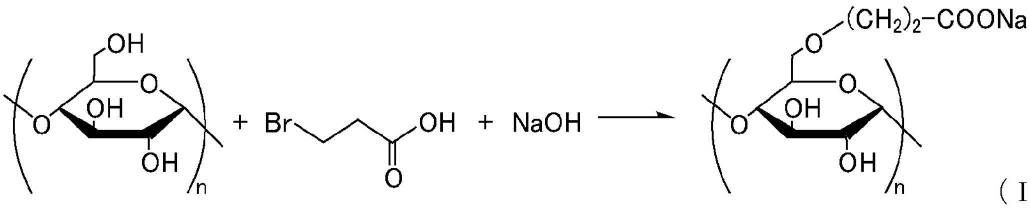

(II)

The reaction of the starch or partially degraded starch with 6-bromohexanoic acid to produce a water-soluble polymer is summarized in formula (III).

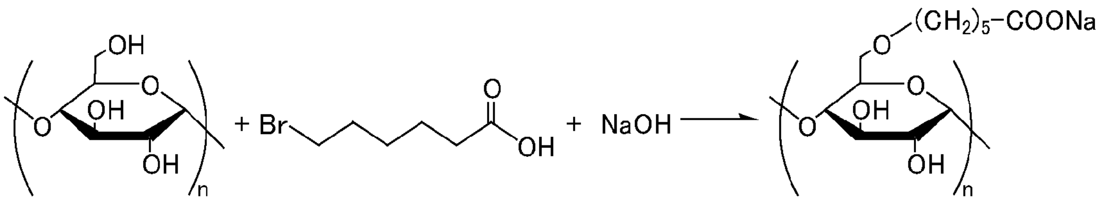

(III)

The reaction of the starch or partially degraded starch with succinic anhydride to produce a water-soluble polymer is summarized in formula (IV).

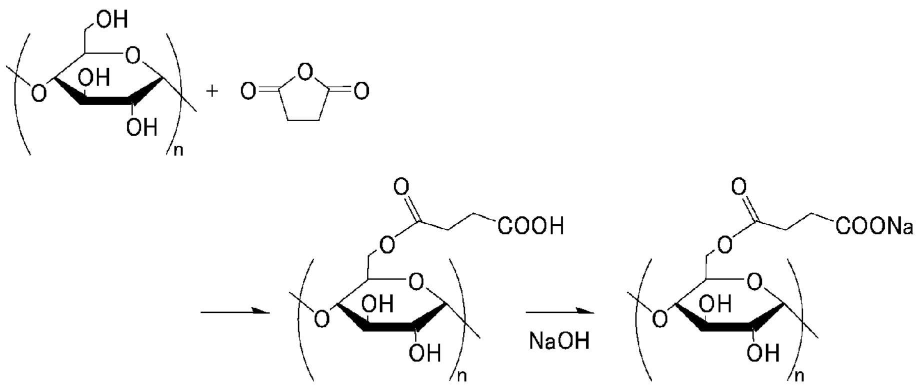


(IV)

The reaction of the starch or partially degraded starch with maleic anhydride to produce a water-soluble polymer is summarized in formula (V).

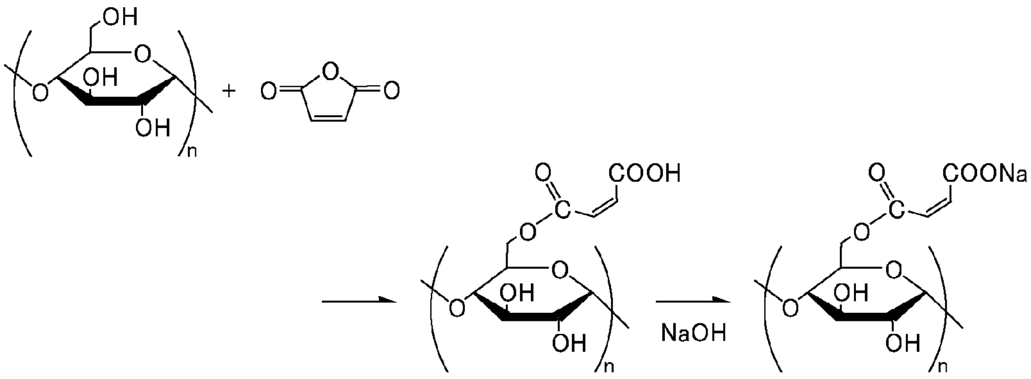


(V)

The reaction of the starch or partially degraded starch with sodium vinylsulfonate to produce a water-soluble polymer is summarized in formula (VI).

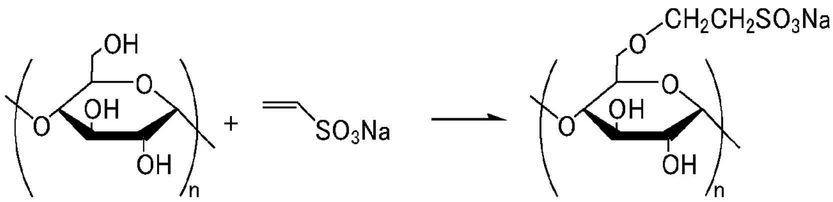

(VI)

Formulas (I) to (VI) show water-soluble polymers in which a sodium salt of an acidic group has been introduced into all hydroxyl groups at the 6-position of the glucose units, but there may remain a hydroxyl group with no acidic group introduced thereinto. There may also be an acidic group that has not been neutralized by the salt. The acidic group can be introduced into the hydroxyl group at any position in the starch or partially degraded starch, and the hydroxyl group may be at any of the 1-, 2-, 3-, 4-, and 6-positions.

When a haloalkyl compound or a haloalkenyl compound is used as the acidic group-containing compound, it is preferable to use 1 to 1.5 equivalents of an alkaline agent relative to the haloalkyl compound or haloalkenyl compound. Examples of the alkaline agent include sodium hydroxide, potassium hydroxide, lithium hydroxide, ammonia, sodium carbonate, and potassium carbonate. The acidic group introduced into the starch or partially degraded starch preferably forms a salt with the sodium, potassium, lithium, ammonia, or the like derived from the alkaline agent. To this end, the alkaline agent is preferably used in an amount required for both the reaction of the haloalkyl compound or haloalkenyl compound with the starch or partially degraded product thereof and the neutralization of the acidic group of the haloalkyl compound or haloalkenyl compound. For example, when chloroacetic acid is used as the acidic group-containing compound, the alkaline agent is preferably used theoretically in an amount of at least 2 equivalents relative to the chloroacetic acid. When sodium chloroacetate is used, since the acidic group has already been neutralized, the alkaline agent is preferably used in an amount of at least 1 equivalent relative to the sodium chloroacetate.

The amount of the acidic group-containing compound to be used can be arbitrarily set depending on the desired total acid value (degree of etherification) of the water-soluble polymer. Usually, the amount is preferably 0.5 to 5.0 equivalents, more preferably 0.5 to 2 equivalents per mole of hydroxyl groups of the starch or partially degraded starch. When a haloalkyl compound such as chloroacetic acid in the form of an aqueous solution is to be reacted, the haloalkyl compound is required in excess of the theoretical amount because the introduction reaction of the acidic group will compete with the hydrolysis reaction of the haloalkyl compound. The amount of the haloalkyl compound to be used in the reaction of the aqueous solution is preferably set to be 5 equivalents or less relative to the theoretical value.

The temperature during the reaction between the starch or partially degraded starch and the acidic group-containing compound is not limited, but it is preferably 0°C to 120°C. The reaction time is not limited, but it is preferably 1 to 24 hours. The reaction may be performed in water, but it may be performed in a solvent mixture of water with an alcohol such as methanol, ethanol, isopropanol, butanol, ethylene glycol, diethylene glycol, propylene glycol, or ethylene glycol monoethyl ether, a glycol ether such as ethylene glycol dimethyl ether, or other solvent. The reaction may also be performed by dispersing powder of the dried partially degraded starch in a hydrophilic solvent, for example, an alcohol such as methanol, ethanol, isopropanol, or butanol, or a glycol ether such as ethylene glycol dimethyl ether. When a solvent mixture is used, the percentage of the solvent other than water is preferably 50% by volume or less based on the solvent mixture. The reactor used may be a reaction vessel or an extruder, for example.

When a haloalkyl compound, such as chloroacetic acid, or a salt thereof is used as the acidic group-containing compound, the temperature during the reaction with the starch or partially degraded starch is not limited, but it is preferably 0°C to 100°C.

In particular, when chloroacetic acid or a salt thereof is used as the haloalkyl compound, the reaction is preferably performed at 25°C to 90°C to prevent hydrolysis due to the water in the reaction solution. The reaction time is preferably a period of time until the haloalkyl compound as a raw material is consumed, more preferably 1 to 12 hours for stability of the haloalkyl compound and process efficiency. The reaction may be performed in water, but it may be performed in a solvent mixture of water with an alcohol such as methanol, ethanol, isopropanol, or butanol, a glycol ether such as ethylene glycol dimethyl ether, or other solvent. The reaction may also be performed by dispersing powder of the dried partially degraded starch in a hydrophilic solvent, for example, an alcohol such as methanol, ethanol, isopropanol, or butanol, or a glycol ether such as ethylene glycol dimethyl ether. When a solvent mixture is used, the percentage of the solvent other than water is preferably 50% by volume or less based on the solvent mixture. The reactor used may be a reaction vessel or an extruder, for example.

Also, when an acid anhydride is used as the acidic group-containing compound, the reaction is allowed to proceed by only mixing and heating the partially degraded starch and the acid anhydride. However, in order to promote the reaction, sodium carbonate, sodium hydroxide, a tertiary amine such as triethylamine, an imidazole such as 2-methylimidazole, a quaternary ammonium salt such as tetrabutylammonium bromide, or a phosphonium salt such as tetrabutylphosphonium bromide may be used as a catalyst. The amount of such a catalyst to be added is preferably 0.1 equivalents or less relative to the acidic group-containing compound. These catalysts may be used alone or in combinations of two or more.

The reaction time is preferably a period of time until the acid anhydride as a raw material is consumed, more preferably 1 to 12 hours. The end point of the reaction can be determined by the measurement of the acid value or IR measurement. The reaction may be performed in water, but the reaction solvent used is preferably an aprotic solvent, such as dimethylsulfoxide, dimethylformamide, dimethylacetamide, or N-methylpyrrolidone, in order to prevent hydrolysis or alcoholysis of the acid anhydride. When a solvent mixture is used, the percentage of solvent other than water is preferably at least 50% by volume based on the solvent mixture. When the reaction is performed with no solvent, the reaction is preferably performed at a temperature equal to or higher than the melting point of the acid anhydride because the acid anhydride can serve as a solvent. When the reaction is performed with a solvent, the reaction temperature is preferably 50°C to 100°C, more preferably 70°C to 90°C. The reactor used may be a reaction vessel or an extruder, for example.

Some of the salts of the acidic groups added to the starch or partially degraded starch are preferably neutralized. The neutralization converts some of the salt-forming acidic groups into free acidic groups. For example, when monochloroacetic acid is used as the acidic group-containing compound described above and sodium hydroxide is used as the alkaline agent, a sodium salt of a carboxyl group is added to the starch or partially degraded starch. By adding an acid thereto, some carboxyl groups can be converted into free carboxylic acids.

Any acid may be used for the neutralization. When the acidic group used is a carboxyl group, the acid is preferably an acid having a pKa equal to or less than that of the carboxyl group. Examples of such acids include hydrochloric acid, sulfuric acid, phosphoric acid, nitric acid, acetic acid, formic acid, and trichloroacetic acid. A strong acid is preferably used for the neutralization of a sulfo group or a phospho group. Examples of the strong acid used include mineral acids such as hydrochloric acid and sulfuric acid, and strongly acidic ion exchange resins. The neutralization can be performed with a known device such as a reaction vessel or an extruder. After the addition of the acid, stirring is preferably performed at 0°C to 50°C for 0.2 to 1 hour for the neutralization reaction. The neutralization reaction is preferably performed at pH 6.8 to 7.2.

In the step of introducing an acidic group or in the subsequent neutralization reaction, a salt may be formed between the halogen derived from the acidic group-containing compound and the metal or ammonia derived from the alkaline agent, and therefore desalting is preferably performed. The desalting may be performed by a washing process including dropping an aqueous solution in which the water-soluble polymer is dissolved in water into a hydrophilic solvent such as methanol, ethanol, isopropanol, acetone, or acetonitrile to reprecipitate the water-soluble polymer, and collecting it by filtration, followed by redispersing and stirring the water-soluble polymer collected by filtration in hydrous methanol (with a water content of about 70% to 90%), and then collecting the particles of the water-soluble polymer by filtration. The desalting may also be performed by treating an aqueous solution of the hydrophilic polymer with a filter having an ultrafiltration membrane. The washing liquid used for desalting may be water or a mixture of water and a hydrophilic organic solvent such as methanol, ethanol, propanol, acetone, or acetonitrile. The desalting is preferably performed until the salt concentration in the water-soluble polymer reaches 1% or less.

In the step of forming an internal cross-linked structure, the water-soluble polymer molecules are cross-linked. The cross-links can be formed with no cross-linking agent, for example, by heat drying the water-soluble polymer, in which some acidic groups are free acidic groups, under hydrous conditions. The temperature during the heat drying is preferably 50°C to 150°C, more preferably 60°C to 130°C. The atmospheric pressure during the heat drying is not limited, and the heat drying may be performed at normal pressure; specifically, it is preferably performed at 0.9 to 1.1 atm. The drying can be performed by any method such as using a drum dryer, a spray dryer, or a Nauta mixer.

The water-soluble polymer may contain a hydrophilic solvent in addition to water during the heat drying. Examples of the hydrophilic solvent include lower aliphatic alcohols such as methanol, ethanol, n-propanol, and isopropanol; ketones such as acetone; ethers such as dioxane, tetrahydrofuran, and methoxy (poly)ethylene glycol; and amides such as ε-caprolactam and N,N-dimethylformamide. The percentage of the hydrophilic solvent other than water based on the total solvent is preferably adjusted depending on the boiling point of the solvent. When the boiling point of the solvent is 100°C or lower, the percentage is preferably 70% by volume or more, whereas when the boiling point of the solvent is higher than 100°C, the percentage is preferably 30% by volume or less. Moreover, the water-soluble polymer at the start of heat drying may be in the form of an aqueous solution or powder containing a hydrous solvent with a water content of 1% by weight or more. When the powder containing the hydrous solvent is dried, the wet ratio of the powder at the start of drying is preferably 1 to 85% by weight, more preferably 20 to 80% by weight, still more preferably 55 to 75% by weight. As used herein, the term "wet ratio" refers to the percentage of the combined amount of water and hydrophilic solvent based on the powder.

In the production of the water-absorbing resin, surface cross-linking may be performed in addition to internal cross-linking. The surface cross-linking can improve the strength of the water-absorbing resin. Examples of the cross-linking agent used for surface cross-linking include epoxy compounds, polyhydric alcohol compounds, polyamine compounds, polyisocyanate compounds, alkylene carbonate compounds, haloepoxy compounds, halohydrin compounds, polyvalent oxazoline compounds, carbodiimide compounds, silane coupling agents, and polyvalent metal compounds.

Examples of the epoxy compounds include glycidyl succinate, sorbitol polyglycidyl ether, trimethylolpropane polyglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol.

Examples of the polyhydric alcohol compounds include ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanediol, trimethylolpropane, diethanolamine, triethanolamine, polyoxypropylene, oxyethylene-oxypropylene block copolymers, pentaerythritol, and sorbitol.

Examples of the polyamine compounds include ethylenediamine, diethylenetriamine, triethylenetetramine, tetraethylenepentamine, pentaethylenehexamine, polyethyleneimine, and inorganic or organic salts (such as azitinium salts) of these polyamine compounds.

Examples of the polyisocyanate compounds include 2,4-tolylene diisocyanate and hexamethylene diisocyanate, and examples of the polyvalent oxazoline compounds include 1,2-ethylenebisoxazoline.

Examples of the alkylene carbonate compounds include 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, and 4,6-dimethyl-1,3-dioxan-2-one.

Examples of the haloepoxy compounds include epichlorohydrin, epibromohydrin, α-methylepichlorohydrin, and polyamine adducts thereof (for example, Kymene® available from Hercules Incorporated).

Other examples of usable known cross-linking agents include waterborne carbodiimide compounds (for example, CARBODILITE available from Nisshinbo Chemical Inc.); silane coupling agents such as γ-glycidoxypropyltrimethoxysilane and γ-aminopropyltriethoxysilane; and polyvalent metal compounds such as hydroxides or chlorides of zinc, calcium, magnesium, aluminum, iron, zirconium, etc.

Preferred among these are epoxy compounds. More preferred are ethylene glycol diglycidyl ether, sorbitol polyglycidyl ether, and glycidyl succinate.

Surface cross-links can be formed by spraying a surface cross-linking agent to the water-absorbing resin and mixing them to cause cross-linking by a known method with a cylindrical mixer, a V-shaped mixer, a ribbon mixer, a screw mixer, a twin-arm mixer, a pulverizing kneader, or the like. A surfactant may be added as necessary during the spraying or mixing.

The water-absorbing resin may further contain other additives, such as a disinfectant, a deodorant, an antimicrobial agent, a fragrance, various inorganic powders, a foaming agent, a pigment, a dye, hydrophilic short fibers, a fertilizer, an oxidizing agent, a reducing agent, water, and salts, in order to impart various functions. The amounts of these additives can be appropriately selected by those skilled in the art.

<Application of absorbent article>

The absorbent article of the present invention may be used as hygiene supplies, medical supplies, civil engineering supplies, food storage supplies, or agricultural and horticultural supplies, for example.

The absorbent article of the present invention can absorb and retain protein-containing fluids. Therefore, the absorbent article may be used in hygiene and medical applications to absorb and retain urine as well as protein-containing fluids such as blood, menstrual blood, and breast milk. Specific examples of such hygiene supplies include disposable

diapers, sanitary products, incontinence pads, breast pads, facial masks, portable toilets, pet toilets, and cat litter. Examples of such medical supplies include hemostatic sponges (medical pads), wound protection materials, wound healing materials, medical waste solidifiers, cold packs, drug delivery system carriers, artificial joints, and poultices.

Examples of food storage supplies include refrigerants, cooling gels, water retention materials, anti-condensation sheets, drip absorbents, and freshness-keeping agents.

Examples of civil engineering supplies include waterstop materials, liquid waste solidifiers, surplus soil solidifiers, and anti-condensation building materials.

Examples of agricultural and horticultural supplies include soil conditioners, soil water-retaining materials, seedling pots, nursery beds, hydroponic supports, fertilizer retention materials, and fertilizer slow-release agents.

Examples of other applications include air fresheners, disposable body warmers, portable toilets, and thickening agents for cosmetics and other applications.

<Disposable diaper, Sanitary napkin>

The absorbent article of the present invention may have any structure suitable for each of the applications as long as it contains the water-absorbing resin described above. In the case where the absorbent article is used as a disposable diaper or a sanitary napkin, the disposable diaper or sanitary napkin includes a stack of a topsheet, an absorbent, and a backsheet stacked in this order.

The topsheet comes into contact with the body of the person wearing the disposable diaper or sanitary napkin. The topsheet preferably has liquid permeability and may include a hydrophilic nonwoven fabric or a porous resin film, for example. The backsheet does not come into contact with the body of the person wearing the disposable diaper or sanitary napkin, but is located on the outside. The backsheet preferably has no liquid permeability and may include a hydrophobic nonwoven fabric, a resin film, or a stack of a nonwoven fabric and a resin film, for example.

The absorbent includes the water-absorbing resin described above. The absorbent may be a formed product obtained by forming the water-absorbing resin into a predetermined shape. The absorbent may be used by wrapping the water-absorbing resin with a core wrap. Examples of the core wrap include paper sheets and liquid-permeable nonwoven fabrics.

In addition to the above-described water-absorbing resin, the absorbent may further contain hydrophilic fibers, for example, natural hydrophilic fibers such as water-absorbing paper, pulp, acetate, tissue, hydrophilic nonwoven fabric, or cotton, cellulose-based natural hydrophilic fibers such as lyocell, rayon, or cupra, petroleum-derived hydrophilic fibers obtained by treating acrylic fibers, or a water-absorbing resin such as a polyacrylic acid-based, polyaspartic acid-based, or starch-acrylonitrile-based resin. The percentage of the water-absorbing resin including the water-soluble polymer, in which an acidic group is introduced into a starch or a partially degraded product thereof, based on the absorbent is preferably 35% by weight or more, more preferably 40% by weight or more, in the case of a disposable diaper, while it is preferably 5% by weight or more, more preferably 10% by weight or more, in the case of a sanitary napkin. Whether used in a disposable diaper or a sanitary napkin, the upper limit is not limited and is 100% by weight or less.

When the absorbent contains hydrophilic fibers, the amount of hydrophilic fibers relative to 100 parts by weight of the water-absorbing resin is preferably 10 to 500 parts by weight, more preferably 20 to 300 parts by weight.

<Recycled water-soluble polymer>

A recycled water-soluble polymer can be obtained by treating the absorbent article or the water-absorbing resin in the absorbent article with an alkali. In the alkali treatment, the absorbent article or the water-absorbing resin in the absorbent article is preferably placed under conditions with a pH of 9.0 or more, more preferably 10.0 or more. The alkali treatment can cleave the cross-linked structure or glucoside bonds of the water-absorbing resin to degrade it into a water-soluble polymer, thereby reducing the environmental burden at the time of disposal.

Non-limiting examples of an alkaline agent used in the alkali treatment include sodium hydroxide, potassium hydroxide, lithium hydroxide, ammonia, sodium carbonate, and potassium carbonate. In the alkali treatment using an alkaline aqueous solution, the concentration of the alkaline agent in the aqueous solution is preferably 0.01 to 20% by weight, more preferably 0.1 to 5% by weight. The alkali treatment may be performed at any temperature, for example, at 5°C to 70°C. The alkali treatment is preferably performed for 1 to 120 minutes. After the alkali treatment, the recycled water-soluble polymer may optionally be subjected to neutralization by addition of an acid, concentration, washing, filtration, and/or other processes. The recycled water-soluble polymer preferably has a molecular weight, dispersity, total acid value, free acid value, or dispersity described above for the water-soluble polymer.

A water-absorbing resin can be obtained by cross-linking the recycled water-soluble polymer. The cross-linking method and cross-linked structure of the recycled water-soluble polymer are preferably as described above for the water-soluble polymer. The water-absorbing resin obtained by cross-linking the recycled water-soluble polymer preferably has physical properties as described above for the water-absorbing resin. The water absorbency under no pressure for ion-exchanged water, water retention rate for ion-exchanged water, water absorbency under no pressure for physiological saline, and water retention rate for physiological saline of the water-absorbing resin obtained by cross-linking the recycled water-soluble polymer are preferably 60% to 140%, more preferably 80% to 120%, still more preferably 90% to 110%, particularly preferably 95% to 105%, of the respective water absorption characteristics of a water-absorbing resin obtained by cross-linking the non-recycled water-soluble polymer.

An absorbent article can be produced from the water-absorbing resin obtained by cross-linking the recycled water-soluble polymer. The structure and application of the absorbent article are as described above.

<Method for recovering hydrophilic fibers>

The method for recovering hydrophilic fibers according to the present invention is a method for recovering hydrophilic fibers from an absorbent article containing a water-absorbing resin and the hydrophilic fibers, the water-absorbing resin including a water-soluble polymer obtained by introducing an acidic group into a starch or a partially degraded product thereof, the method including the step of degrading the water-absorbing resin by treating a part or the entirety of the water absorbent article with an alkali.

The water-absorbing resin, the hydrophilic fibers, and the absorbent article may be as described above. In the step of degrading the water-absorbing resin, the object to be treated with an alkali may be the entirety or a part of the water absorbent article. When a part of the water absorbent article is to be treated with an alkali, the object to be treated contains the water-absorbing resin and the hydrophilic fibers. The alkali treatment conditions may be as described for the recycled water-soluble polymer. When a part or the entirety of the water absorbent article is treated with an alkali, the water-absorbing resin is degraded and dissolved so that the hydrophilic fibers can be obtained as solid residues. The hydrophilic fibers obtained as solid residues in the step of degrading the water-absorbing resin may then be dried, optionally defibrated or fibrillated, and used like virgin pulp for the production of an absorbent article such as a disposable diaper. Moreover, the recovered hydrophilic fibers can be used in a water-absorbing article as well as in applications that generally use hydrophilic fibers, such as an industrial wiper, a liquid waste absorbent material, and a filter.

## EXAMPLES

Hereinafter, the present invention will be described with reference to examples, but the present invention is not limited thereto. Hereinafter, "part(s)" and "%" mean "part(s) by weight" and "% by weight", respectively, unless otherwise specified.

### (1) Materials used

#### (1-1) Starch raw material

Corn starch
Waxy corn starch
Tapioca

#### (1-2) Hydrolase

α-amylase (Spitase® HK/R available from Nagase ChemteX Corporation), 12,200 units/g

Amylomaltase: *Thermus thermophilus* was aerobically cultured. Then, a homogenate extract of the collected bacterial cells was centrifuged, and the supernatant was used as a crude enzyme solution. The crude enzyme solution was subjected to column chromatography in a conventional manner and then electrophoretically purified to a single product. The resulting sample was used as a purified enzyme solution.

Here, the activity of the amylomaltase was measured by the following method. A reaction liquid containing 10% by weight maltotriose, a 50 mM sodium acetate buffer (pH 6.0), and the enzyme was incubated at 60°C for 20 minutes. Thereafter, the reaction was stopped by heating at 100°C for 10 minutes. The amount of glucose in the reaction liquid was measured by a glucose oxidase method. For the unit amount of amylomaltase, the activity of amylomaltase that produces 1 μmol of glucose per minute was defined as one unit.

### (2) Production of partially degraded starch (Production Examples 1 to 6)

Partially degraded starches were produced in the following manner. Here, the weight average molecular weight

of each partially degraded starch obtained was determined by aqueous size exclusion chromatography relative to a calibration curve of molecular weight vs. elution time prepared using pullulans with known molecular weights. Table 1 shows the weight average molecular weight and dispersity of each partially degraded starch.

(Production Example 1) Partially degraded starch derived from corn starch

Corn starch was suspended in city water to a concentration of 30% by weight, and then a 1 N aqueous sodium hydroxide solution was added thereto to adjust the pH to 6.0 to obtain a starch milk. To the starch milk were added 2.0 units of the crude enzyme solution of amylomaltase per gram of the starch solids. The mixture was stirred at room temperature for 30 minutes and then reacted at 80°C for six hours while stirring to prepare a liquefied starch. The obtained partially degraded starch had a weight average molecular weight of 6,000,000 and a dispersity of 30.2.

(Production Example 2) Partially degraded starch derived from corn starch

Corn starch was suspended in city water to a concentration of 30% by weight, and then a 1 N aqueous sodium hydroxide solution was added thereto to adjust the pH to 6.0 to obtain a starch milk. To the starch milk were added 0.1 units of the crude enzyme solution of amylomaltase per gram of the starch solids. The mixture was stirred at room temperature for 30 minutes and then reacted at 90°C for 4.5 hours while stirring to prepare a liquefied starch. The obtained partially degraded starch had a weight average molecular weight of 2,260,000 and a dispersity of 14.9.

(Production Example 3) Partially degraded starch derived from tapioca

The concentration of tapioca was adjusted to 15% by weight. Thereto were added 0.1 units of the crude enzyme solution of amylomaltase per gram of the starch solids. The mixture was stirred at room temperature for 30 minutes and then reacted at 80°C for four hours while stirring to prepare a liquefied starch. The obtained partially degraded starch had a weight average molecular weight of 1,180,000 and a dispersity of 22.6.

(Production Example 4) Partially degraded starch derived from tapioca

A liquefied starch was prepared by the same procedure as in Production Example 3, except that the reaction time was 12 hours. The obtained partially degraded starch had a weight average molecular weight of 440,000 and a dispersity of 17.3.

(Production Example 5) Partially degraded starch derived from waxy corn starch

Waxy corn starch was suspended in city water to a concentration of 15% by weight, and then a 1 N aqueous sodium hydroxide solution was added thereto to adjust the pH to 6.0 to obtain a starch milk. To the starch milk were added 0.5 units of $\alpha$-amylase per gram of the starch solids. The mixture was stirred at room temperature for 30 minutes, and then reacted at 100°C for 20 minutes while stirring to prepare a liquefied starch. The obtained partially degraded starch had a weight average molecular weight of 610,000 and a dispersity of 35.2. An aqueous solution of the partially degraded starch was dried in vacuum at 80°C. The resulting dried product was pulverized to collect a dry powder product of 1 mm pass. The moisture content of the dry powder product was 4.8%.

(Production Example 6) Partially degraded starch derived from corn starch

Corn starch was suspended in city water to a concentration of 30% by weight, and then a 1 N aqueous sodium hydroxide solution was added thereto to adjust the pH to 6.0 to obtain a starch milk. To the starch milk were added 0.2 units of the crude enzyme solution of amylomaltase per gram of the starch solids. The mixture was stirred at room temperature for 30 minutes and then reacted at 90°C for 4.5 hours while stirring to prepare a liquefied starch. The obtained partially degraded starch had a weight average molecular weight of 1,910,000 and a dispersity of 10.1.

[Table 1]

| | Production Example 1 | Production Example 2 | Production Example 3 | Production Example 4 | Production Example 5 | Production Example 6 |
|---|---|---|---|---|---|---|
| Mw | 6,000,000 | 2,260,000 | 1,180,000 | 440,000 | 610,000 | 1,910,000 |
| Dispersity | 30.2 | 14.9 | 22.6 | 17.3 | 35.2 | 10.1 |

(3) Production of water-soluble polymer

(Production Example 7)

A 140 g portion of a 30% by weight aqueous solution of the partially degraded starch produced in Production Example 1 (0.78 mol of hydroxyl groups of the partially degraded starch) was charged into a 500 ml separable flask equipped with a stirrer, a thermometer, and a condenser tube. Next, 37.6 g of a 48.8% aqueous sodium hydroxide solution (0.46 mol, 0.59 equivalents relative to the hydroxyl groups of the partially degraded starch) was charged into the flask and stirred at 60°C or lower until the solution became completely homogeneous. After confirming that the solution was homogeneous, an aqueous solution prepared by dissolving 20.6 g of monochloroacetic acid (0.44 mol, 0.8 equivalents relative to the hydroxyl groups of the partially degraded starch) in 5.2 g of ion-exchanged water was charged dropwise at 50°C to 60°C over 30 minutes. After charging the aqueous monochloroacetic acid solution, the temperature was adjusted to 45°C to 50°C, and the mixture was stirred for 12 hours. The end point of the reaction was determined by sampling the reaction liquid and measuring the chloride ion content in the reaction liquid by potentiometric titration with a 0.01 N aqueous silver nitrate solution, and was defined as the point at which the chloride ion content reached at least 98% of the calculated chloride ion content of 3.8%, which corresponds to the chloride ion content when all the monochloroacetic acid has reacted. In this production example, the chlorine content was 3.75%.

After completion of the reaction, the reaction liquid was diluted with 600 g of ion-exchanged water. The diluted reaction liquid was cooled to room temperature and added to 5000 mL of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1000 mL of hydrous methanol with a methanol/water ratio = 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01 N aqueous silver nitrate solution, and the washing process was repeated until the chlorine content was less than 1%. The resulting water-soluble polymer had a total acid value of 128 mg KOH/g, a degree of etherification calculated from the total acid value of 0.45, a weight average molecular weight of 30,310,000, and a dispersity of 25.7.

(Production Example 8)

A 200 g portion of a 30% by weight aqueous solution of the partially degraded starch produced in Production Example 2 (1.11 mol of hydroxyl groups of the partially degraded starch) was charged into a 500 ml separable flask equipped with a stirrer, a thermometer, and a condenser tube. Next, 79.4 g of a 48.8% aqueous sodium hydroxide solution (0.96 mol, 0.86 equivalents relative to the hydroxyl groups of the partially degraded starch) was charged into the flask and stirred at 60°C or lower until the solution became completely homogeneous. After confirming that the solution was homogeneous, 89.9 g of crystals of 6-bromohexanoic acid (0.46 mol, 0.4 equivalents relative to the hydroxyl groups of the partially degraded starch) were charged little by little at 50°C to 60°C over 30 minutes. After charging the 6-bromohexanoic acid, the temperature was adjusted to 45°C to 50°C, and the mixture was stirred for 10 hours. The end point of the reaction was determined by sampling the reaction liquid and measuring the bromide ion content in the reaction liquid by potentiometric titration with a 0.01 N aqueous silver nitrate solution, and was defined as the point at which the bromide ion content reached at least 98% of the calculated bromide ion content of 9.8%, which corresponds to the bromide ion content when all the 6-bromohexanoic acid has reacted. In this production example, the bromine content was 10.1%.

After completion of the reaction, the reaction liquid was diluted with 250 g of ion-exchanged water. The diluted reaction liquid was cooled to room temperature and added to 1600 mL of ethanol over about 30 minutes to reprecipitate a water-soluble polymer. After adding all the reaction liquid, the mixture was stirred for 30 minutes, and the water-soluble polymer dispersed in ethanol was subjected to solid-liquid separation by filtration under reduced pressure.

Subsequently, in order to remove the sodium bromide contained in the water-soluble polymer, the collected water-soluble polymer was redispersed in 500 mL of hydrous ethanol with an ethanol/water ratio of 90/10 (by volume) and then stirred and washed at room temperature for 30 minutes, followed by performing solid-liquid separation by filtration under reduced pressure to collect the water-soluble polymer again. The bromine content of the collected water-soluble polymer was measured by potentiometric titration with a 0.01 N aqueous silver nitrate solution, and the washing process was repeated until the bromide ion content was less than 1%. The resulting water-soluble polymer had a total acid value of 133 mg KOH/g, a degree of etherification calculated from the total acid value of 0.55, a weight average molecular weight of 10,420,000, and a dispersity of 24.7.

(Production Example 9)

A 200 g portion of a 30% by weight aqueous solution of the partially degraded starch produced in Production Example 3 (1.11 mol of hydroxyl groups of the partially degraded starch) was charged into a 500 ml separable flask equipped with a stirrer, a thermometer, and a condenser tube. Next, 47.8 g of a 48.8% aqueous sodium hydroxide solution (0.58 mol, 1.1 equivalents relative to the hydroxyl groups of the partially degraded starch) was charged into the flask and stirred at 60°C or lower until the solution became completely homogeneous. After confirming that the solution was homogeneous, an aqueous solution prepared by dissolving 77.7 g of sodium monochloroacetate (0.67 mol, 1.2 equivalents relative to the hydroxyl groups of the partially degraded starch) in 111 g of water was charged little by little at 50°C to 60°C over 30 minutes. After charging the aqueous sodium monochloroacetate solution, the temperature was adjusted to 90°C to 95°C, and the mixture was stirred for five hours. The end point of the reaction was determined by sampling the reaction liquid and measuring the chloride ion content in the reaction liquid by potentiometric titration with a 0.01 N aqueous silver nitrate solution, and was defined as the point at which the chloride ion content reached at least 98% of the calculated chloride ion content of 5.4%, which corresponds to the chloride ion content when all the sodium monochloroacetate has reacted. In this production example, the chlorine content was 5.3%.

After completion of the reaction, the reaction liquid was cooled to room temperature and added to 2000 mL of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1000 mL of methanol and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01 N aqueous silver nitrate solution, and the washing process was repeated until the chloride ion content was less than 1%. The resulting water-soluble polymer had a total acid value of 152 mg KOH/g, a degree of etherification calculated from the total acid value of 0.56, a weight average molecular weight of 8,680,000, and a dispersity of 18.3.

(Production Example 10)

A water-soluble polymer was obtained by performing a reaction and post-treatments as in Production Example 9, except that the partially degraded starch was changed to the partially degraded starch produced in Production Example 4. The water-soluble polymer had a total acid value of 148 mg KOH/g, a degree of etherification calculated from the total acid value of 0.54, a weight average molecular weight of 3,070,000, and a dispersity of 9.5.

(Production Example 11)

A 50 g portion of powder (moisture content: 4.8%) of the partially degraded starch produced in Production Example 5 (0.88 mol of hydroxyl groups of the partially degraded starch) and 140 g of water were charged into a 1000 ml separable flask equipped with a stirrer, a thermometer, and a condenser tube. Next, 94.7 g of a 48.8% aqueous sodium hydroxide solution (1.2 mol, 1.3 equivalents relative to the hydroxyl groups of the partially degraded starch) was charged into the flask and stirred at 60°C or lower until the solution became completely homogeneous. After confirming that the solution was homogeneous, an aqueous solution prepared by dissolving 154 g of sodium monochloroacetate (1.3 mol, 1.5 equivalents relative to the hydroxyl groups of the partially degraded starch) in 300 g of water was charged little by little at 50°C to 60°C over 30 minutes. After charging the aqueous sodium monochloroacetate solution, the temperature was adjusted to 90°C to 95°C, and the mixture was stirred for five hours. The end point of the reaction was determined by sampling the reaction liquid and measuring the chloride ion content in the reaction liquid by potentiometric titration with a 0.01 N aqueous silver nitrate solution, and was defined as the point at which the chloride ion content reached at least 98% of the calculated chloride ion content of 6.3%, which corresponds to the chloride ion content when all the sodium monochloroacetate has reacted. In this production example, the chlorine content was 6.3%.

After completion of the reaction, the reaction liquid was cooled to room temperature and added to 5000 mL of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 2000 mL of methanol and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01 N aqueous silver nitrate solution, and the washing process was repeated until the chloride ion content was less than

1%. The resulting water-soluble polymer had a total acid value of 192 mg KOH/g, a degree of etherification calculated from the total acid value of 0.76, a weight average molecular weight of 2,300,000, and a dispersity of 22.3.

(Production Example 12)

A water-soluble polymer was obtained by performing a reaction and post-treatments as in Production Example 7, except that the partially degraded starch was changed to 140 g of the partially degraded starch produced in Production Example 6, the amount of the 48.8% aqueous sodium hydroxide solution was changed to 55.6 g (0.68 mol, 0.87 equivalents relative to the hydroxyl groups of the partially degraded starch), the amount of the monochloroacetic acid was changed to 30.5 g (0.32 mol, 0.4 equivalents relative to the hydroxyl groups of the partially degraded starch), and the amount of ion-exchanged water used to dissolve the monochloroacetic acid was changed to 8 g. The water-soluble polymer had a total acid value of 137 mg KOH/g, a degree of etherification calculated from the total acid value of 0.49, a weight average molecular weight of 11,890,000, and a dispersity of 15.3.

(4) Production of water-absorbing resin

(Production Example 13)

A 170 g portion of the water-soluble polymer obtained in Production Example 7 (product wet with hydrous methanol; wet ratio: 69%) was introduced into a 1000 mL beaker, and 550 mL of hydrous methanol with a methanol/water ratio = 80/20 (by volume) was further added to disperse the water-soluble polymer. To this dispersion, 5.8 mL of a 1 N aqueous hydrochloric acid solution was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to free acid. The collected water-soluble polymer was wet crystals containing a hydrous alcohol. The wet crystals were transferred to a petri dish, introduced into a fan dryer set at 70°C, and dried for 20 hours to perform cross-linking treatment. The solids obtained after the treatment were pulverized in a mortar and sieved through sieves with openings of 150 μm and 850 μm to collect water-absorbing resin particles having a particle size of 150 to 850 μm.

(Production Example 14)

A 170 g portion of the water-soluble polymer obtained in Production Example 8 (product wet with hydrous ethanol; wet ratio: 61%) was introduced into a 1000 mL beaker, and 500 mL of ethanol was further added to disperse the water-soluble polymer. To this dispersion, 82.2 mL of a 1 N aqueous hydrochloric acid solution was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to free acid. The collected water-soluble polymer was wet crystals containing a hydrous alcohol. The wet crystals were transferred to a petri dish, introduced into a fan dryer set at 150°C, and dried for one hour to perform cross-linking treatment. The solids obtained after the treatment were pulverized in a mortar and sieved through sieves with openings of 150 μm and 850 μm to collect water-absorbing resin particles having a particle size of 150 to 850 μm.

(Production Example 15 to 21)

Water-absorbing resins were produced by the same procedure as in Example 13, except that the raw materials and the amounts thereof charged were changed as shown in Table 2.

[Table 2]

| | Production Example 13 | Production Example 14 | Production Example 15 | Production Example 16 | Production Example 17 | Production Example 18 | Production Example 19 | Production Example 20 | Production Example 21 |
|---|---|---|---|---|---|---|---|---|---|
| Partially hydrolyzed starch | Production Example 1 | Production Example 2 | Production Example 3 | Production Example 3 | Production Example 4 | Production Example 5 | Production Example 5 | Production Example 6 | Production Example 1 |
| Water-soluble polymer | Production Example 7 | Production Example 8 | Production Example 9 | Production Example 9 | Production Example 10 | Production Example 11 | Production Example 11 | Production Example 12 | Production Example 7 |
| Water-soluble polymer (g) | 170 | 170 | 45 | 45 | 45 | 45 | 45 | 40 | 40 |
| Water-soluble polymer wet ratio (%) | 69 | 61 | 67 | 67 | 66 | 68 | 68 | 71 | 69 |
| Hydrous methanol (ml) | 550 | Ethanol 500 | 120 | 120 | 120 | 120 | 120 | 120 | 100 |
| 1N Hydrochloric acid (ml) | 5.8 | 82.2 | 4.4 | 5.9 | 3.1 | 4.3 | 5.8 | 11.6 | 6.5 |
| Drying temperature (°C) | 70 | 150 | 70 | 70 | 70 | 70 | 70 | 70 | 70 |
| Duration of drying (time) | 20 | 1 | 12 | 12 | 12 | 12 | 12 | 20 | 12 |
| Solid content (%) | 96.1 | 97.0 | 98.2 | 97.2 | 98.3 | 97.4 | 97.8 | 97.5 | 96.4 |

(Comparative Production Example 1)

Production of water-absorbing resin including sodium polyacrylate

An amount of 155 parts by mass (2.15 molar parts) of acrylic acid, 0.62 parts by mass (0.0024 molar parts) of pentaerythritol triallyl ether as an internal cross-linking agent, and 340.27 parts by mass of deionized water were stirred and mixed while maintaining the temperature at 1°C. Nitrogen was allowed to flow into the mixture to adjust the amount of dissolved oxygen to 0.1 ppm or less. Then, 0.31 parts by mass of a 1% by mass aqueous hydrogen peroxide solution, 1.16 parts by mass of a 1% by mass aqueous ascorbic acid solution, and 2.325 parts by mass of a 0.5% by mass aqueous solution of 2,2'-azobis[2-methyl-N-(2-hydroxyethyl)propionamide] were added and mixed to start polymerization. After the temperature of the mixture reached 85°C, the polymerization was carried out for about 10 hours at 85 ± 2°C to obtain a hydrogel. Next, while shredding 502.27 parts by mass of the hydrogel with a mincing machine ("12VR-400K", Royal), 128.42 parts by mass of a 48.5% by mass aqueous sodium hydroxide solution was added to and mixed with the hydrogel, and then 3 parts by mass of a 1% by mass aqueous solution of ethylene glycol diglycidyl ether was added and mixed to obtain gel shreds. Further, the gel shreds were dried in a ventilated band dryer (200°C, wind speed: 5 m/s) to obtain dried products. The dried products were ground in a juicer mixer ("Osterizer Blender", Oster) and then sieved through sieves with openings of 150 μm and 710 μm to adjust the particle size to 150 μm to 710 um. Thus, dried particles were obtained.

(5) Production of disposable diaper

(Examples 1 to 7)

A 100 mm × 133 mm water-absorbing resin/pulp mixture absorbent (basis weight of pulp: 0.0375 g/cm$^2$, basis weight of water-absorbing resin: 0.03 g/cm$^2$) was produced by mixing 4 g of a water-absorbing resin and 5 g of ground wood pulp using a batch-type air-laid machine. The water-absorbing resin/pulp mixture absorbent was sandwiched between tissue paper from above and below, and then the mixture absorbent was pressed back and forth 10 times by a 15 kg roller to obtain an absorbent layer. The absorbent layer was sandwiched between a thermally bonded nonwoven fabric (18 gsm) and a polyethylene sheet, and the circumference was fixed with a duct tape to obtain a disposable diaper sample. Table 3 shows the water-absorbing resin used.

(Comparative Example 1)

A disposable diaper was produced as in Examples 1 to 7, except that the water-absorbing resin used was the water-absorbing resin produced in Comparative Production Example 1.
The absorption rate, wet back amount, and spreading length of the disposable diapers were measured by the methods described later. Table 3 shows the results.

[Table 3]

| | Partially degraded starch | Water-soluble polymer | Water-absorbing resin | Starch raw material | Acidic group | Absorption rate (sec) | | | Wet back amount (WB) (g) | | | | Spreading length (cm) |
|---|---|---|---|---|---|---|---|---|---|---|---|---|---|
| | | | | | | First | Second | Third | First | Second | Third | Total | |
| Example 1 | Production Example 1 | Production Example 7 | Production Example 13 | Corn starch | Carboxymethyl group | 2.1 | 11.4 | 41.3 | 4.7 | 5.1 | 15.3 | 25.1 | 11.8 |
| Example 2 | Production Example 2 | Production Example 8 | Production Example 14 | Corn starch | Carboxypentyl group | 1.7 | 10.8 | 24.8 | 5.4 | 9.2 | 16.8 | 31.4 | 11.8 |
| Example 3 | Production Example 3 | Production Example 9 | Production Example 15 | Tapioca | Carboxymethyl group | 4.1 | 38.0 | 58.5 | 4.1 | 9.0 | 13.3 | 26.4 | 12.5 |
| Example 4 | Production Example 3 | Production Example 9 | Production Example 16 | Tapioca | Carboxymethyl group | 3.3 | 29.1 | 38.2 | 1.4 | 10.5 | 14.2 | 26.1 | 12.5 |
| Example 5 | Production Example 4 | Production Example 10 | Production Example 17 | Tapioca | Carboxymethyl group | 3.6 | 36.9 | 55.0 | 3.7 | 11.0 | 14.6 | 29.3 | 13.5 |
| Example 6 | Production Example 5 | Production Example 11 | Production Example 18 | Waxy corn starch | Carboxymethyl group | 3.3 | 33.5 | 56.2 | 4.2 | 8.1 | 19.9 | 32.2 | 13.5 |
| Example 7 | Production Example 5 | Production Example 11 | Production Example 19 | Waxy corn starch | Carboxymethyl group | 3.6 | 35.1 | 48.4 | 4.6 | 11.4 | 16.7 | 32.7 | 13.0 |
| Comparative Example 1 | - | - | - | - | - | 1.6 | 29.8 | 69.9 | 2.3 | 7.7 | 10.4 | 20.4 | 12.2 |

* In Comparative Example 1, the water-absorbing resin of Comparative Production Example 1 including Na polyacrylate was used.

The disposable diapers of Examples 1 to 7 exhibited practically sufficient performance as disposable diapers in terms of all properties: absorption rate, wet back amount, and spreading length. The disposable diapers of some examples exhibited performance much higher than that of the disposable diaper of Comparative Example 1 using conventional sodium polyacrylate.

(6) Production of sanitary napkin

(Examples 8 to 10)

A 50 mm × 70 mm water-absorbing resin absorbent (basis weight of water-absorbing resin: 0.0143 g/$cm^2$) was produced from 0.5 g of a water-absorbing resin using a batch-type air-laid machine. The absorbent was sandwiched between tissue paper from above and below, and then the absorbent was pressed back and forth 10 times by a 15 kg roller to obtain an absorbent layer. The absorbent layer was sandwiched between polyethylene sheets, and the circumference was fixed with a duct tape to obtain a sanitary napkin sample. Table 4 shows the water-absorbing resin used.

(Comparative Example 2)

A sanitary napkin was produced as in Examples 8 to 10, except that the water-absorbing resin used was the water-absorbing resin of Comparative Production Example 1.
The absorption rate, wet back amount, absorption amount, and water retention amount of the sanitary napkins were measured by the methods described later. Table 4 shows the results.

[Table 4]

| | Partially degraded starch | Water-soluble polymer | Water-absorbing resin | Starch raw material | Acidic group | Absorption rate (sec) | Wet back amount (WB) (g) | Absorption amount (g/g) | Water retention amount -150 G (g/g) |
|---|---|---|---|---|---|---|---|---|---|
| Example 8 | Production Example 6 | Production Example 12 | Production Example 20 | Corn starch | Carboxymethyl group | 17.8 | 0.2 | 11.2 | 7.3 |
| Example 9 | Production Example 1 | Production Example 7 | Production Example 21 | Corn starch | Carboxymethyl group | 19.4 | 0.2 | 8.8 | 6.6 |
| Example 10 | Production Example 2 | Production Example 8 | Production Example 14 | Corn starch | Carboxypentyl group | 14.5 | 0.3 | 12.6 | 7.4 |
| Comparative Example 2 | - | - | - | - | - | 72.9 | 2.3 | 6.5 | 5.7 |

* In Comparative Example 2, the water-absorbing resin of Comparative Production Example 1 including Na polyacrylate was used.

The sanitary napkins of Examples 8 to 10 exhibited higher performance than the sanitary napkin of Comparative Example 2 using conventional sodium polyacrylate in terms of all properties: absorption rate, wet back amount, absorption amount, and water retention amount.

(7) Method for evaluating water-soluble polymer (7-1) Total acid value of water-soluble polymer

About 0.3 g of the water-soluble polymer was precisely weighed into a 100 mL beaker and dissolved in 40 mL of ion-exchanged water. This aqueous solution was set in a potentiometric titrator (AT-610 available from Kyoto Electronics Manufacturing Co., Ltd.) equipped with glass electrodes (C-171 available from Kyoto Electronics Manufacturing Co., Ltd.). A sample in which all groups formed a sodium salt would have a potential of approximately 30 mV or less at this stage, and therefore a 1 N aqueous hydrochloric acid solution was added until the potential reached 320 mV or more to convert all carboxylic acid groups in the water-soluble polymer into the free acid form (resulting in an excess of hydrochloric acid). After confirming that the potential was 320 mV or more, neutralization titration was performed with a 0.1 N aqueous sodium hydroxide solution. In this titration, two inflection points were detected, with the first inflection point being near 220 mV and the second inflection point being near 0 to -30 mV. The former is the neutralization point of the excess hydrochloric acid in the sample and the latter is the neutralization point of the carboxylic acid in the water-soluble polymer. Thus, the total acid value is calculated by the following equation 1:

$$\text{Total acid value (mg KOH/g)} = [\{(Vb - Va) \times 0.1 \times fa \times 56.11\} \div Sa]/(1 - wr) \quad \text{(Equation 1)}$$

wherein

Va represents the volume (ml) of the 0.1 N aqueous sodium hydroxide solution consumed up to the first inflection point;
Vb represents the volume (ml) of the 0.1 N aqueous sodium hydroxide solution consumed up to the second inflection point;
fa represents the titer of the 0.1 N aqueous sodium hydroxide solution;
Sa represents the amount of the sample collected; and
wr represents the wet ratio of the water-soluble polymer measured by the method described later.

(7-2) Wet ratio of water-soluble polymer

The term "wet ratio" refers to the rate (%) of reduction in weight relative to the initial weight of the sample when the sample is dried at a drying temperature of 130°C and measured with a halogen moisture meter. In the examples, 0.5 to 1.0 g of the water-soluble polymer was set in a halogen moisture meter HC103 available from Mettler Toledo and measured at a drying temperature of 130°C and a switch-off criterion of 1 mg/50 seconds in % MC mode (a mode that displays MC value = (initial weight of sample - dry weight of sample) ÷ initial weight of sample × 100). The displayed MC value was taken as the wet ratio.

(7-3) Degree of etherification of water-absorbing resin

$$\text{Degree of etherification} = (162 \times TAV) \div (56100 - 80 \times TAV)$$

In the equation, TAV represents the total acid value (unit: mg KOH/g) of the water-soluble polymer.

(8) Method for evaluating disposable diaper

(8-1) Absorption rate

The disposable diaper is placed on a horizontal plane with the topsheet facing upwards. A measurement acrylic plate (weight: 200 g) with a cylinder is put on the central part of the diaper. Then, 25 g of physiological saline (salt concentration: 0.9% by weight) is poured into the cylinder at the central part all at once. The time taken for the physiological saline in the cylinder to be absorbed into the disposable diaper is measured as the absorption rate.

(8-2) Wet back amount

The disposable diaper after the absorption rate test is left for five minutes. Then, the measurement acrylic plate

with a cylinder is removed, an about 20 g filter paper (the weight is precisely weighed and denoted as W1 (g)) is put on the disposable diaper, and further a 3.5 kg weight is put on the filter paper to apply pressure for three minutes. After three minutes, the filter paper is removed, and its weight is measured (W2 (g)). The wet back amount is determined using the following equation.

$$\text{Wet back amount (g)} = W2 - W1$$

(8-3) Spreading length

The operations described in the sections (8-1) Absorption rate and (8-2) Wet back amount are further repeated two times for a total of three measurements. The length (maximum length) of the physiological saline spread on the disposable diaper is measured.

(9) Method for evaluating sanitary napkin

(9-1) Absorption amount

The sanitary napkin was immersed in 1000 mL of milk for one hour without stirring and then hung for 10 minutes for drainage. The weight (h5) of the sanitary napkin was measured, and the absorption amount was determined using the following equation. Here, the temperatures of the milk used and the measurement atmosphere were 25°C ± 2°C.

$$\text{Absorption amount (g/sheet)} = (h5) - (h6)$$

Here, (h6) denotes the weight of the sanitary napkin before the immersion.

(9-2) Water retention amount

After the absorption amount test, the sanitary napkin was placed in a centrifuge and centrifuged and dehydrated at 150 G for 120 seconds to remove excess milk. The weight (h7) of the sanitary napkin was measured, and the water retention amount was determined using the following equation.

$$\text{Water retention amount (g/sheet)} = (h7) - (h6)$$

(9-3) Absorption rate

A sanitary napkin is placed on a horizontal plane with the topsheet facing upwards. A measurement acrylic cylindrical jig (weight: 200 g) is put on the central part of the napkin. Then, 5 g of milk is poured into the cylinder at the central part all at once. The time taken for the milk in the cylinder to be absorbed into the sanitary napkin was measured as the absorption rate.

(9-4) Wet back amount

The sanitary napkin after the absorption rate test is left for five minutes. Then, the measurement acrylic cylindrical jig is removed, an about 20 g filter paper (the weight is precisely weighed and denoted as W1 (g)) is put on the sanitary napkin, and further a 0.5 kg weight is put on the filter paper to apply pressure for three minutes. After three minutes, the filter paper is removed, and its weight is measured (W2 (g)). The wet back amount is determined using the following equation.

$$\text{Wet back amount (g)} = W2 - W1$$

(10) Degradation and recycle of water-absorbing resin

(10-1) Production of disposable diaper

(10-1-1) Production of partially degraded starch derived from tapioca (Production Example 22)

Tapioca starch was suspended in city water to a concentration of 30% by weight, and then a 1 N aqueous sodium

hydroxide solution was added thereto to adjust the pH to 6.0 to obtain a starch milk. To the starch milk were added 8.0 units of the crude enzyme solution of amylomaltase per gram of the starch solids. The mixture was stirred at room temperature for 30 minutes and then reacted at 80°C for five hours while stirring to prepare a liquefied starch. The obtained partially degraded starch had a weight average molecular weight of 2,820,000 and a dispersity of 9.82.

(10-1-2) Production of water-soluble polymer (Production Example 23)

A 140 g portion of a 30% by weight aqueous solution of the partially degraded starch of Production Example 22 (0.78 mol of hydroxyl groups of the partially degraded starch) was charged into a 500 ml separable flask equipped with a stirrer, a thermometer, and a condenser tube. Next, 37.6 g of a 48.8% aqueous sodium hydroxide solution (0.46 mol, 0.59 equivalents relative to the hydroxyl groups of the partially degraded starch) was charged into the flask and stirred at 60°C or lower until the solution became completely homogeneous. After confirming that the solution was homogeneous, an aqueous solution prepared by dissolving 20.6 g of monochloroacetic acid (0.44 mol, 0.8 equivalents relative to the hydroxyl groups of the partially degraded starch) in 5.2 g of ion-exchanged water was charged dropwise at 60°C to 70°C over 30 minutes. After charging the aqueous monochloroacetic acid solution, the temperature was adjusted to 80°C to 90°C, and the mixture was stirred for one hour. The end point of the reaction was determined by sampling the reaction liquid and measuring the chloride ion content in the reaction liquid by potentiometric titration with a 0.01 N aqueous silver nitrate solution, and was defined as the point at which the chloride ion content reached at least 98% of the calculated chloride ion content of 3.8%, which corresponds to the chloride ion content when all the monochloroacetic acid has reacted. In this production example, the chlorine content was 3.81%.

After completion of the reaction, the reaction liquid was diluted with 600 g of ion-exchanged water. The diluted reaction liquid was cooled to room temperature and added to 5000 mL of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1000 mL of hydrous methanol with a methanol/water ratio = 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01 N aqueous silver nitrate solution, and the washing process was repeated until the chlorine content was less than 1%. The resulting water-soluble polymer had a total acid value of 131.7 mg KOH/g, a weight average molecular weight of 9,420,000, and a dispersity of 15.3.

(10-1-3) Production of water-absorbing resin (Production Example 24)

A 170 g portion of the water-soluble polymer obtained in Production Example 23 (product wet with hydrous methanol; wet ratio: 68%) was introduced into a 1000 mL beaker, and 550 mL of hydrous methanol with a methanol/water ratio = 80/20 (by volume) was further added to disperse the water-soluble polymer. To this dispersion, 10.5 mL of a 1 N aqueous hydrochloric acid solution was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to free acid. The collected water-soluble polymer was wet crystals containing a hydrous alcohol. The wet crystals were transferred to a petri dish, introduced into a fan dryer set at 70°C, and dried for 20 hours to perform cross-linking treatment. The solids obtained after the treatment were pulverized in a mortar and sieved through sieves with openings of 150 μm and 850 μm to collect water-absorbing resin particles having a particle size of 150 to 850 μm.

Production of water-absorbing resin including sodium polyacrylate (Comparative Production Example 2)

An amount of 155 parts by mass (2.15 molar parts) of acrylic acid, 0.62 parts by mass (0.0024 molar parts) of pentaerythritol triallyl ether as an internal cross-linking agent, and 340.27 parts by mass of deionized water were stirred and mixed while maintaining the temperature at 1°C. Nitrogen was allowed to flow into the mixture to adjust the amount of dissolved oxygen to 0.1 ppm or less. Then, 0.31 parts by mass of a 1% by mass aqueous hydrogen peroxide solution, 1.16 parts by mass of a 1% by mass aqueous ascorbic acid solution, and 2.325 parts by mass of a 0.5% by mass aqueous solution of 2,2'-azobis[2-methyl-N-(2-hydroxyethyl)propionamide] were added and mixed to start polymerization. After the temperature of the mixture reached 85°C, the polymerization was carried out for about 10 hours at 85 ± 2°C to obtain a hydrogel. Next, while shredding 502.27 parts by mass of the hydrogel with a mincing machine ("12VR-400K", Royal), 128.42 parts by mass of a 48.5% by mass aqueous sodium hydroxide solution was added to and mixed with the hydrogel, and then 3 parts by mass of a 1% by mass aqueous solution of ethylene glycol diglycidyl ether was added and mixed to obtain gel shreds. Further, the gel shreds were dried in a ventilated band dryer (200°C, wind speed: 5 m/s) to obtain dried

products. The dried products were ground in a juicer mixer ("Osterizer Blender", Oster) and then sieved through sieves with openings of 150 μm and 710 μm to adjust the particle size to 150 μm to 710 μm. Thus, dried particles were obtained.

(10-1-4) Production of disposable diaper (Example 11)

An amount of 5.0 g of the water-absorbing resin produced in Production Example 24 was put on 5.0 g of a water-absorbing pulp sheet with a size of 10 cm × 40 cm, and the water-absorbing pulp sheet was folded in two to a size of 10 cm × 20 cm. Thus, a water-absorbing resin/pulp mixture absorbent was produced. The water-absorbing resin/pulp mixture absorbent was sandwiched between tissue paper from above and below, and then the mixture absorbent was pressed back and forth 10 times by a 15 kg roller to obtain an absorbent layer. The absorbent layer was sandwiched between a topsheet (air-through nonwoven fabric (18 gsm) and a backsheet (polyethylene sheet), and the circumference was fixed with a duct tape to obtain a disposable diaper sample.

(10-1-5) Production of disposable diaper (Comparative Example 3)

A disposable diaper was produced as in Example 11, except that the water-absorbing resin produced in Production Example 24 was replaced by the water-absorbing resin produced in Comparative Production Example 2.

(10-1-6) Absorption amount of disposable diaper

An amount of 40 mL of physiological saline was applied to the disposable diaper of Example 11 or Comparative Example 3 three times at five-minute intervals to allow the disposable diaper to absorb a total of 120 mL of physiological saline. Both disposable diapers could absorb a total of 120 mL of physiological saline. Here, the temperatures of the physiological saline used and the measurement atmosphere were 25°C ± 2°C.

(10-1-7) Water retention amount of disposable diaper

After the absorption amount test, the disposable diaper was placed in a centrifuge and centrifuged and dehydrated at 150 G for 120 seconds to remove excess physiological saline. Then, the weight of the disposable diaper was measured. The weight of the disposable diaper before the water absorption was subtracted from the measured weight to determine the water retention amount. The result of Example 11 was 106.5 g/sheet, and the result of Comparative Example 3 was 111.9 g/sheet.

(10-2) Degradation of water-absorbing resin (Production Example 25)

After the water retention amount measurement, the water-absorbing resin/pulp mixture absorbent was taken out from the disposable diaper of Example 11 and then immersed in 1000 mL of an aqueous sodium hydroxide solution (concentration: 0.5% by weight) at 25°C ± 2°C for 30 minutes.

After the solid residues of the absorbent were subjected to washing with water and centrifugal dehydration, they were measured for ash content in accordance with JIS P 8251:2003 "Paper, board and pulps-Determination of residue (ash) on ignition at 525 degrees C". If all the water-absorbing resin remains in the solid residues of the absorbent, the ash content to be measured is presumably about 6% due to the presence of the sodium in the aqueous sodium hydroxide solution used in the production of the water-soluble polymer.

The ash content of an unused pulp was measured for comparison and found to be -0.7%. The theoretical ash content of the unused pulp is 0, and the measured ash content is presumed to be within the margin of error. Meanwhile, the measured ash content of the solid residues of the absorbent was 0.8%, which was comparable to that of the unused pulp. The results demonstrate that almost all the water-absorbing resin in the absorbent was degraded by the alkali treatment.

After the immersion of the water-absorbing resin/pulp mixture absorbent in the aqueous sodium hydroxide solution, the degraded product of the water-absorbing resin could be present as a recycled water-soluble polymer in the aqueous solution. The recovered recycled water-soluble polymer was analyzed by GPC and IR and compared with the unused water-soluble polymer produced in Production Example 23, and FIGS. 1 and 2 show the results. Moreover, Table 5 shows the molecular weights and the dispersities. As shown in Table 5 and FIGS. 1 and 2, the molecular weight distribution and chemical structure of the recycled water-soluble polymer in the degraded product of the water-absorbing resin were comparable to those of the unused water-soluble polymer.

[Table 5]

| | Weight average molecular weight (Mw) | Number average molecular weight (Mn) | Dispersity (Mw/Mn) |
|---|---|---|---|
| Unused water-soluble polymer | 9,581,546 | 567,980 | 16. 87 |
| Water-soluble polymer in degraded product | 9,552,635 | 493,158 | 19.37 |

(10-3) Recycle of water-absorbing resin from degraded product

The aqueous solution containing the recycled water-soluble polymer derived from the degraded product obtained in Production Example 25 (weight: 427 g, solid content: 1.4% by weight, alkali content: 26.1 meg/g) was neutralized to a pH of 7 to 8 by adding hydrochloric acid dropwise. The solution was evaporated to remove water to a solid content of 10% by weight using an evaporator. The residues were reprecipitated in methanol and subjected to suction filtration under reduced pressure to collect 10.5 g of a wet recycled water-soluble polymer (solid content: 39.6%, net: 4.2 g). To the collected wet recycled water-soluble polymer was added 300 mL of hydrous methanol with a methanol/water ratio = 80/20 (by volume) to disperse the recycled water-soluble polymer. To this dispersion, 0.8 mL of a 1 N aqueous hydrochloric acid solution was gradually added with a measuring pipette while stirring with a magnetic stirrer. After adding the hydrochloric acid, the mixture was stirred for 15 minutes and subjected to suction filtration under reduced pressure to collect the recycled water-soluble polymer partially neutralized to free acid. The collected recycled water-soluble polymer was transferred to a petri dish, introduced into a fan dryer set at 70°C, and dried for 20 hours to perform cross-linking treatment. The solids obtained after the treatment were pulverized in a mortar and sieved through sieves with openings of 150 μm and 850 μm to collect water-absorbing resin particles having a particle size of 150 to 850 μm. Table 6 shows the water absorption characteristics of this water-absorbing resin and a water-absorbing resin produced from the unused water-soluble polymer by the same method.

[Table 6]

| Material of water-absorbing resin | Water absorbency under no pressure for ion-exchanged water (g/g) | Water retention rate for ion-exchanged water (g/g) | Water absorbency under no pressure for physiological saline (g/g) | Water retention rate for physiological saline (g/g) |
|---|---|---|---|---|
| Unused water-soluble polymer | 284.1 | 208.8 | 51.1 | 46.7 |
| Recycled water-soluble polymer | 390.1 | 204.4 | 53.9 | 50.3 |

As shown in Table 6, the water-absorbing resin produced again from the recycled water-soluble polymer, which was the degraded product of the water-absorbing resin, also exhibited sufficient water absorption characteristics.

(10-4) Recovery of pulp (Production Examples 26 and 27, Comparative Production Examples 3 and 4)

The solid residues (recovered pulp) of the absorbent recovered by the alkali treatment of the absorbent in Production Example 25 were dried by a fan dryer at 120°C and then fibrillated. The fibrillated recovered pulp or a virgin pulp was used in combination with the absorbing resin shown in Table 7 to produce a disposable diaper having the same structure as in Example 11. The water absorption rate and wet back amount of the disposable diaper were evaluated. Table 7 shows the results.

[Table 7]

| | Material of model absorbent | | Water absorption rate (sec) | | | | Wet back amount (g/sheet) | | | |
|---|---|---|---|---|---|---|---|---|---|---|
| | Water-absorbing resin | Pulp | First | Second | Third | Total | First | Second | Third | Total |
| Production Example 26 | Production Example 24 | Recovered pulp (Production Example 25) | 4 | 16 | 42 | 62 | 11 | 12 | 14 | 37 |
| Production Example 27 | Comparative Production Example 2 | Recovered pulp (Production Example 25) | 4 | 57 | 92 | 153 | 6 | 10 | 10 | 26 |
| Comparative Production Example 3 | Production Example 24 | Virgin pulp | 2 | 13 | 50 | 65 | 10 | 12 | 15 | 36 |
| Comparative Production Example 4 | Comparative Production Example 2 | Virgin pulp | 3 | 44 | 100 | 147 | 7 | 9 | 10 | 26 |

As shown in Table 7, the performance obtained with the recovered pulp was comparable to that obtained with virgin pulp. It is demonstrated that the recovered pulp can be used like virgin pulp for the production of a water-absorbing article.

## Claims

1. An absorbent article, comprising a water-absorbing resin comprising a water-soluble polymer obtained by introducing an acidic group into a starch or a partially degraded product thereof.

2. The absorbent article according to claim 1,
wherein the acidic group is a carboxyalkyl group, a carboxyalkenyl group, or a sulfoalkyl group.

3. The absorbent article according to claim 1 or 2,
wherein the starch or partially degraded product thereof has at least one of a weight average molecular weight (Mw) of 7,500,000 or less or a dispersity (weight average molecular weight (Mw)/number average molecular weight (Mn)) of 5 or more.

4. The absorbent article according to any one of claims 1 to 3,
wherein the water-soluble polymer has a weight average molecular weight (Mw) of 500,000 to 40,000,000 as determined by aqueous size exclusion chromatography using pullulan standards.

5. The absorbent article according to any one of claims 1 to 4,
wherein the water-absorbing resin has at least one of the following characteristics:

   (a) a water absorbency under no pressure for ion-exchanged water is 100 to 500 g/g;
   (b) a water retention rate for ion-exchanged water is 80 to 300 g/g;
   (c) a water absorbency under no pressure for physiological saline is 10 to 70 g/g; and
   (d) a water retention rate for physiological saline is 5 to 60 g/g.

6. The absorbent article according to any one of claims 1 to 5, which is at least one selected from the group consisting of hygiene supplies, medical supplies, food storage supplies, civil engineering supplies, and agricultural and horticultural supplies.

7. The absorbent article according to claim 6,

wherein the hygiene supplies are at least one selected from the group consisting of disposable diapers, sanitary products, incontinence pads, breast pads, portable toilets, and pet toilets.

**8.** The absorbent article according to claim 6,
wherein the medical supplies are at least one selected from the group consisting of hemostatic sponges, wound protection materials, wound healing materials, medical waste solidifiers, cold packs, drug delivery system carriers, and artificial joints.

**9.** A recycled water-soluble polymer, obtained by treating the absorbent article according to any one of claims 1 to 5 with an alkali.

**10.** A water-absorbing resin that is a crosslinked product of the recycled water-soluble polymer according to claim 9.

**11.** A water-absorbing article, comprising the water-absorbing resin according to claim 10.

**12.** A method for recovering hydrophilic fibers from an absorbent article containing a water-absorbing resin and the hydrophilic fibers, the water-absorbing resin comprising a water-soluble polymer obtained by introducing an acidic group into a starch or a partially degraded product thereof,
the method comprising the step of degrading the water-absorbing resin by treating a part or an entirety of the water absorbent article with an alkali.

FIG.1

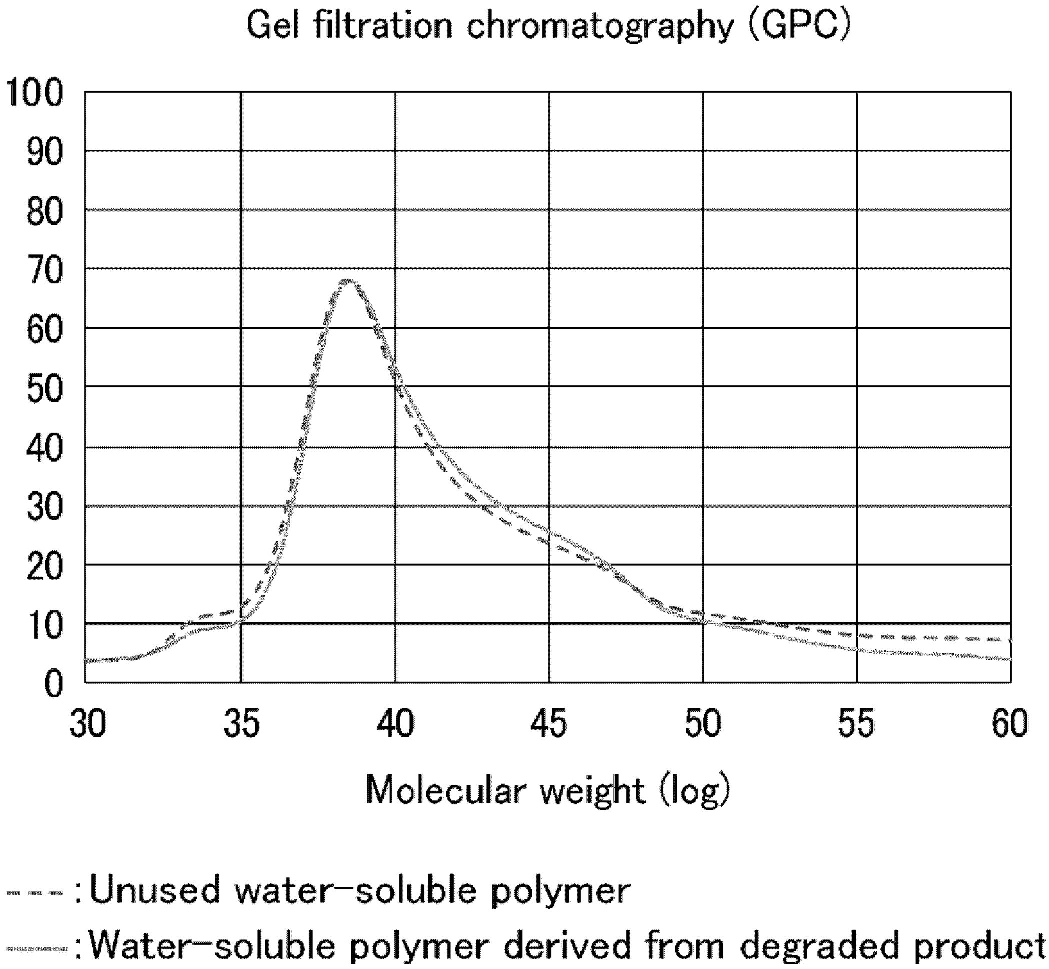
Gel filtration chromatography (GPC)
100
90
80
70
60
50
40
30
20
10
0
30
35
40
45
50
55
60
Molecular weight (log)
: Unused water-soluble polymer
: Water-soluble polymer derived from degraded product

FIG.2

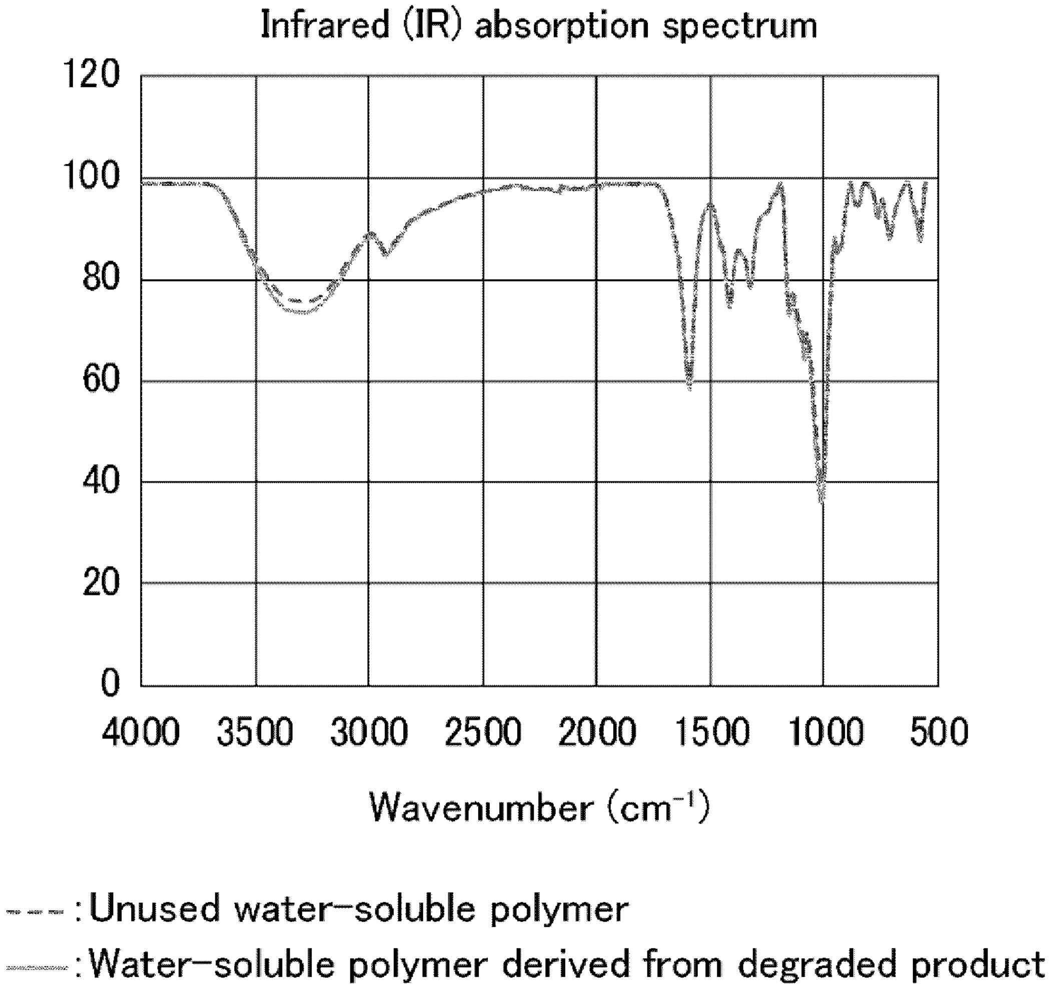
Infrared (IR) absorption spectrum
120
100
80
60
40
20
0
4000
3500
3000
2500
2000
1500
1000
500
Wavenumber ($cm^{-1}$)
: Unused water-soluble polymer
: Water-soluble polymer derived from degraded product

## INTERNATIONAL SEARCH REPORT

International application No.
**PCT/JP2023/024166**

**A. CLASSIFICATION OF SUBJECT MATTER**

***C08B 31/10***(2006.01)i; ***A61F 13/53***(2006.01)i; ***A61K 47/36***(2006.01)i; ***A61L 27/20***(2006.01)i; ***B01J 20/26***(2006.01)i; ***A23L 5/00***(2016.01)i; ***A61L 15/22***(2006.01)i
FI: B01J20/26 D; A23L5/00 G; A23L5/00 Z; A61F13/53 300; A61K47/36; A61L15/22 100; A61L27/20; C08B31/10

According to International Patent Classification (IPC) or to both national classification and IPC

**B. FIELDS SEARCHED**

Minimum documentation searched (classification system followed by classification symbols)

C08B31/10; A61F13/53; A61K47/36; A61L27/20; B01J20/26; A23L5/00; A61L15/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2010-504414 A (ARCHER DANIELS MIDLAND COMPANY) 12 February 2010 (2010-02-12)<br>paragraphs [0002], [0003], [0004], [0011], [0062], examples | 1-8 |
| Y | paragraphs [0002], [0003], [0004], [0011], [0062], examples | 9-12 |
| Y | US 2007/0111194 A1 (PREENTEC AG) 17 May 2007 (2007-05-17)<br>paragraph [0036] | 9-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 August 2023** | **05 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/024166**

| Patent document cited in search report | Publication date (day/month/year) | Patent family member(s) | Publication date (day/month/year) |
|---|---|---|---|
| JP 2010-504414 A | 12 February 2010 | US 2013/0296548 A1<br>paragraphs [0003], [0004], [0009], [0086], examples<br>EP 2066699 A1<br>CA 2664392 A1<br>CN 101541836 A<br>MX 2009003252 A<br>AU 2007302586 A1<br>KR 10-2012-0117944 A<br>BR PI0717171 A | |
| US 2007/0111194 A1 | 17 May 2007 | EP 1694420 A1<br>AT 463294 T | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5079354 A
- JP 2010504414 T
- JP 2007222704 A


### Non-patent literature cited in the description


- *Synthesis*, 1989, vol. 12, 949-950